# EUROPEAN PATENT SPECIFICATION

(11) **EP 4 014 888 B1**
(45) Date of publication and mention of the grant of the patent: **06.11.2024**
(21) Application number: 20852377.9
(22) Date of filing: 17.07.2020
(51) Int. Cl.: A61B 8/14, A61B 8/00, A61B 8/08, H04N 7/18

(54) **ULTRASONIC SYSTEM AND METHOD FOR CONTROLLING ULTRASONIC SYSTEM**
ULTRASCHALLSYSTEM UND VERFAHREN ZUR STEUERUNG EINES ULTRASCHALLSYSTEMS
SYSTÈME ULTRASONORE ET PROCÉDÉ DE COMMANDE DE SYSTÈME ULTRASONORE

(30) Priority: 15.08.2019 JP 2019149058
(43) Date of publication of application: 22.06.2022
(73) Proprietor: FUJIFILM Corporation, Tokyo 106-8620 (JP)
(72) Inventor: HATTORI, Masato, Ashigarakami-gun, Kanagawa 258-8538 (JP); TSUBOTA, Keiji, Ashigarakami-gun, Kanagawa 258-8538 (JP); INOUE, Tomoki, Ashigarakami-gun, Kanagawa 258-8538 (JP)
(74) Representative: Dehns Germany Partnerschaft mbB
(86) International application number: PCT/JP2020/027756
(87) International publication number: WO 2021/029180

(56) References cited:
- JP-A- 2011 183 056
- JP-A- 2011 183 056
- US-A1- 2019 008 486
- US-A1- 2019 008 486
- US-A1- 2019 038 260

## Description

### BACKGROUND OF THE INVENTION

### 1. Field of the Invention

The present invention relates to an ultrasound system and a method of controlling an ultrasound system, and in particular, to an ultrasound system that displays an ultrasound image on a head-mounted display and a method of controlling an ultrasound system.

### 2. Description of the Related Art

Hitherto, in a medical field, an ultrasound diagnostic apparatus using an ultrasound image has come into practical use. In general, this kind of ultrasound diagnostic apparatus has an ultrasound probe that incorporates a transducer array, and an apparatus body connected to the ultrasound probe. The ultrasound probe transmits ultrasonic waves toward a subject and receives ultrasound echoes from the subject, and the apparatus body electrically processes reception signals to generate an ultrasound image.

In such an ultrasound diagnostic apparatus, usually, a monitor on which the ultrasound image is often disposed at a position away from the ultrasound probe, such as a bedside, and thus, an operator needs to alternately move a line of sight between the ultrasound probe at hand and the monitor. Accordingly, to reduce the movement of the line of sight of the operator, for example, an ultrasound diagnostic apparatus comprising a so-called head-mounted display as disclosed in JP2011-183056A has been developed.
Document US 2019/008486 A1 for example describes an ultrasound probe comprising a housing; an acoustic module disposed inside the housing, and configured to transmit an ultrasound signal to an object and receive an echo signal reflected from the object; an image processor disposed in a rear direction of the acoustic module inside the housing, electrically connected to the acoustic module, and configured to generate ultrasound image data from the echo signal received from the acoustic module; a first insulating wall disposed between the acoustic module and the image processor inside the housing; a first heat sink member disposed in a rear direction of the image processor inside the housing; and at least one anisotropic heat conductive member passing through the first insulating wall to connect the acoustic module with the first heat sink member, and configured such that a heat conductivity thereof in a lengthwise direction of the housing is greater than a heat conductivity thereof in a direction perpendicular to the lengthwise direction of the housing to transfer heat of the acoustic module to the first heat sink member.

In general, it is known that, in ultrasound diagnosis using an ultrasound diagnostic apparatus as disclosed in JP2011-183056A, a given level or higher of skill is needed to accurately recognize a part in a subject rendered in an ultrasound image by confirming the ultrasound image. Furthermore, it is known that the image quality of the generated ultrasound image significantly depends on the skill involving the hands of an operator.

Here, for example, in a case where an ultrasound image is captured at a remote location other than a hospital, such as home care, an operator who operates an ultrasound probe to capture an ultrasound image may be different from an observer who observes the captured ultrasound image to perform diagnosis.

In this case, since the operator normally needs to operate the ultrasound probe to capture an ultrasound image of an intended part in a subject while confirming the obtained ultrasound image personally, in particular, in a case where the level of skill of the operator is low, the operator may hardly determine whether or not the intended part of the subject is accurately observed. The operator having a low level of skill may not operate the ultrasound probe using appropriate skill involving the hands, and an ultrasound image with low image quality is obtained. The observer confirms the ultrasound image captured by the operator of the ultrasound diagnostic apparatus to perform diagnosis; however, since the observer cannot recognize a state in which the operator captures the ultrasound image, in particular, in a case where the ultrasound image is captured by the operator having a low level of skill, the observer may hardly accurately determine whether or not the captured ultrasound image is captured by appropriate skill involving the hands.

### SUMMARY

The present invention refers to ultrasound systems and to methods of controlling an ultrasound system with the features of the present independent claims. The present invention has been accomplished to solve such a problem in the related art, and an object of the present invention is to provide an ultrasound system and a method of controlling an ultrasound system capable of obtaining an appropriate ultrasound image and improving accuracy of ultrasound diagnosis even though an ultrasound image is captured at a remote location.

To achieve the above-described object, there is disclosed a first ultrasound system comprising an ultrasound probe, a head-mounted display, and an external apparatus, in which the ultrasound probe includes a transducer array, a transmission and reception circuit that transmits an ultrasonic wave from the transducer array and generates a sound ray signal based on a reception signal acquired by the transducer array, an ultrasound image generation unit that generates an ultrasound image based on the sound ray signal generated by the transmission and reception circuit, and a probe-side wireless communication unit that wirelessly transmits the ultrasound image, the head-mounted display includes a camera unit that acquires a view image obtained by imaging a scanning point of the ultrasound probe in a subject, and a head-mounted display-side wireless communication unit that wirelessly transmits the view image acquired by the camera unit, and the external apparatus includes an external wireless communication unit that is wirelessly connected to at least the head-mounted display-side wireless communication unit, an external monitor, and a display controller that displays the ultrasound image wirelessly transmitted from the ultrasound probe and the view image wirelessly transmitted from the head-mounted display on the external monitor.

The external wireless communication unit may be wirelessly connected to both the probe-side wireless communication unit and the head-mounted display-side wireless communication unit, and the probe-side wireless communication unit may wirelessly transmit the ultrasound image to both the head-mounted display and the external apparatus.

The probe-side wireless communication unit may wirelessly transmit the ultrasound image to the head-mounted display, and the head-mounted display-side wireless communication unit may wirelessly transmit the ultrasound image wirelessly transmitted from the probe-side wireless communication unit and the view image acquired by the camera unit to the external apparatus.

The external apparatus includes an image synchronization unit that synchronizes the ultrasound image and the view image with each other.

The head-mounted display may include a head-mounted display-side monitor, and the ultrasound image may be displayed on the head-mounted display-side monitor.

In this case, it is preferable that the head-mounted display includes an image synchronization unit that synchronizes the ultrasound image and the view image with each other.

The external apparatus may include an input device, the external wireless communication unit may wirelessly transmit external input information input through the input device, to the head-mounted display-side wireless communication unit, and the external input information is displayed on the head-mounted display-side monitor.

Wireless communication of voice data may be performed between the head-mounted display-side wireless communication unit and the external wireless communication unit in two directions.

There is disclosed a method of controlling an ultrasound system including an ultrasound probe, a head-mounted display, and an external apparatus, the method comprising, at the ultrasound probe, transmitting an ultrasonic wave from the transducer array of the ultrasound probe and generating a sound ray signal based on a reception signal acquired by the transducer array, generating an ultrasound image based on the generated sound ray signal, and wirelessly transmitting the ultrasound image; at the head-mounted display, acquiring a view image obtained by imaging a scanning point of the ultrasound probe in a subject, and wirelessly transmitting the acquired view image, and at the external apparatus, displaying the ultrasound image wirelessly transmitted from the ultrasound probe and view image wirelessly transmitted from the head-mounted display on the external monitor.

There is disclosed a second ultrasound system comprising an ultrasound probe, a head-mounted display, and an external apparatus, in which the ultrasound probe includes a transducer array, a transmission and reception circuit that transmits an ultrasonic wave from the transducer array and generates a sound ray signal based on a reception signal acquired by the transducer array, a reception data generation unit that generates reception data before imaging by executing signal processing on the sound ray signal generated by the transmission and reception circuit, and a probe-side wireless communication unit that wirelessly transmits the reception data, the head-mounted display includes a camera unit that acquires a view image obtained by imaging a scanning point of the ultrasound probe in a subject, and a head-mounted display-side wireless communication unit that wirelessly transmits the view image acquired by the camera unit, and the external apparatus includes an external wireless communication unit that is wirelessly connected to at least the head-mounted display-side wireless communication unit, an external monitor, and a display controller that displays an ultrasound image generated based on the reception data wirelessly transmitted from the ultrasound probe and the view image wirelessly transmitted from the head-mounted display on the external monitor.

The external wireless communication unit may be wirelessly connected to both the probe-side wireless communication unit and the head-mounted display-side wireless communication unit, and the probe-side wireless communication unit may wirelessly transmit the reception data to both the head-mounted display and the external apparatus.

The probe-side wireless communication unit may wirelessly transmit the reception data to the head-mounted display, and the head-mounted display-side wireless communication unit may wirelessly transmit the reception data wirelessly transmitted from the probe-side wireless communication unit and the view image acquired by the camera unit to the external apparatus.

The external apparatus may include an image processing unit that generates the ultrasound image based on the reception data wirelessly transmitted from the probe-side wireless communication unit.

The probe-side wireless communication unit may wirelessly transmit the reception data to the head-mounted display, the head-mounted display may include an image processing unit that generates the ultrasound image based on the reception data wirelessly transmitted from the probe-side wireless communication unit, and the head-mounted display-side wireless communication unit may wirelessly transmit the ultrasound image generated by the image processing unit and the view image acquired by the camera unit to the external apparatus.

The external apparatus includes an image synchronization unit that synchronizes the ultrasound image and the view image with each other.

The head-mounted display may include a head-mounted display-side monitor, and the ultrasound image may be displayed on the head-mounted display-side monitor.

In this case, it is preferable that the head-mounted display includes an image synchronization unit that synchronizes the ultrasound image and the view image with each other.

The external apparatus may include an input device, the external wireless communication unit may wirelessly transmit external input information input through the input device, to the head-mounted display-side wireless communication unit, and the external input information is displayed on the head-mounted display-side monitor.

Wireless communication of voice data may be performed between the head-mounted display-side wireless communication unit and the external wireless communication unit in two directions.

There is disclosed a method of controlling an ultrasound system including an ultrasound probe, a head-mounted display, and an external apparatus, the method comprising, at the ultrasound probe, transmitting an ultrasonic wave from the transducer array of the ultrasound probe and generating a sound ray signal based on a reception signal acquired by the transducer array, generating reception data before imaging by executing signal processing on the generated sound ray signal, and wirelessly transmitting the reception data, at the head-mounted display, acquiring a view image obtained by imaging a scanning point of the ultrasound probe in a subject, and wirelessly transmitting the acquired view image, and at the external apparatus, displaying an ultrasound image generated based on the reception data wirelessly transmitted from the ultrasound probe and the view image wirelessly transmitted from the head-mounted display on the external monitor.

The ultrasound probe includes the ultrasound image generation unit that generates the ultrasound image, and the probe-side wireless communication unit that wirelessly transmits the ultrasound image, the head-mounted display includes the camera unit that acquires the view image obtained by imaging the scanning point of the ultrasound probe, and the head-mounted display-side wireless communication unit that wirelessly transmits the view image acquired by the camera unit, and the external apparatus includes the external wireless communication unit that is wirelessly connected to at least the head-mounted display-side wireless communication unit, the external monitor, and the display controller that displays the ultrasound image wirelessly transmitted from the ultrasound probe and the view image wirelessly transmitted from the head-mounted display on the external monitor. Therefore, it is possible to obtain an appropriate ultrasound image and to improve accuracy of ultrasound diagnosis even in a case where an ultrasound image is captured at a remote location.

### BRIEF DESCRIPTION OF THE DRAWINGS

Fig. 1 is a block diagram showing the configuration of an ultrasound system according to Embodiment 1 of the present invention.
Fig. 2 is a block diagram showing the internal configuration of a transmission and reception circuit in Embodiment 1 of the present invention.
Fig. 3 is a diagram showing an example of an HMD in Embodiment 1 of the present invention.
Fig. 4 is a diagram schematically showing an ultrasound image that is displayed on an HMD-side monitor of the HMD in Embodiment 1 of the present invention.
Fig. 5 is a diagram schematically showing an example of an external apparatus in Embodiment 1 of the present invention.
Fig. 6 is a diagram schematically showing an example of a cursor displayed on a view image on an external monitor in a modification example of Embodiment 1 of the present invention.
Fig. 7 is a diagram schematically showing an example of the cursor displayed on the HMD-side monitor in the modification example of Embodiment 1 of the present invention.
Fig. 8 is a block diagram showing the configuration of an HMD in another modification example of Embodiment 1 of the present invention.
Fig. 9 is a block diagram showing the configuration of an external apparatus in another modification example of Embodiment 1 of the present invention.
Fig. 10 is a block diagram showing the configuration of an ultrasound system according to Embodiment 2 of the present invention.
Fig. 11 is a block diagram showing the configuration of an ultrasound system according to Embodiment 3 of the present invention.
Fig. 12 is a block diagram showing the configuration of an ultrasound system according to Embodiment 4 of the present invention.
Fig. 13 is a block diagram showing the configuration of an ultrasound system according to Embodiment 5 of the present invention.
Fig. 14 is a diagram schematically showing an example of ultrasound image and a view image that are displayed on an external apparatus in Embodiment 5 of the present invention.
Fig. 15 is a block diagram showing the configuration of an ultrasound system according to Embodiment 6 of the present invention.

### DESCRIPTION OF THE PREFERRED EMBODIMENTS

Hereinafter, embodiments of the present invention will be described referring to the accompanying drawings.

The description of components described below is provided based on a representative embodiment of the present invention, but the present invention is not limited to such an embodiment.

In the specification, a numerical range represented using "to" means a range including numerical values before and after "to" as a lower limit value and an upper limit value.

In the specification, the terms "same" and "identical" include an error range allowed in the technical field.

### Embodiment 1

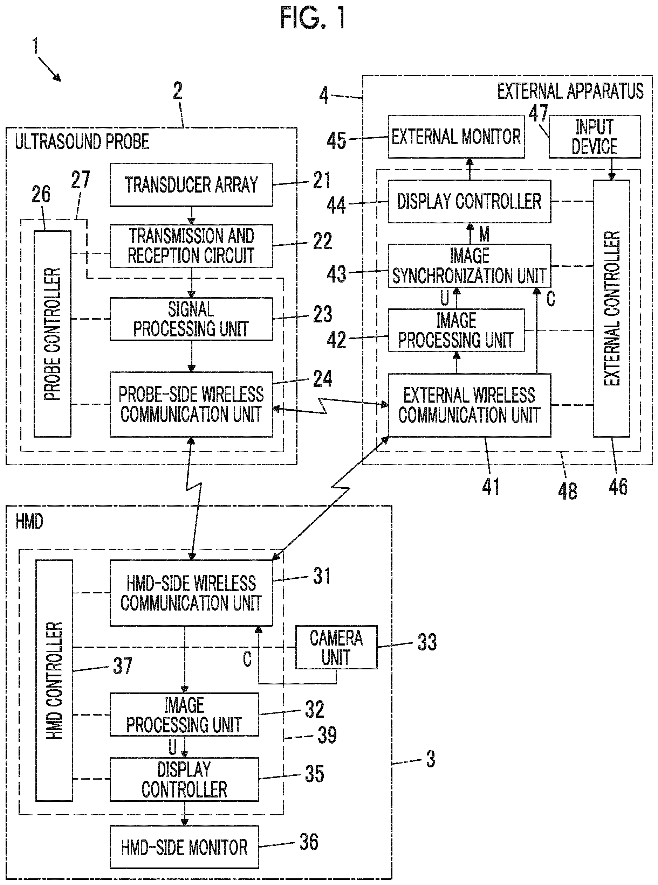

Fig. 1 shows the configuration of an ultrasound system 1 according to Embodiment 1 of the present invention. The ultrasound system 1 comprises an ultrasound probe 2, a head-mounted display (HMD) 3, and an external apparatus 4. The HMD 3 and the external apparatus 4 are connected to the ultrasound probe 2 by wireless communication, and the HMD 3 and the external apparatus 4 are connected to each other by wireless communication.

The ultrasound probe 2 comprises a transducer array 21, and a transmission and reception circuit 22, a signal processing unit 23, and a probe-side wireless communication unit 24 are sequentially connected to the transducer array 21. The probe-side wireless communication unit 24 is connected to the HMD 3 and the external apparatus 4 by wireless communication. Though not shown, the signal processing unit 23 configures a reception data generation unit.

A probe controller 26 is connected to the transmission and reception circuit 22, the signal processing unit 23, and the probe-side wireless communication unit 24. The signal processing unit 23, the probe-side wireless communication unit 24, and the probe controller 26 configure a probe-side processor 27.

The HMD 3 comprises an HMD-side wireless communication unit 31 that is connected to the ultrasound probe 2 and the external apparatus 4 by wireless communication, and an image processing unit 32, a display controller 35, and an head-mounted display-side monitor (HMD-side monitor) 36 are sequentially connected to the head-mounted display-side wireless communication unit (HMD-side wireless communication unit) 31. Furthermore, the HMD 3 comprises a camera unit 33, and the camera unit 33 is connected to the HMD-side wireless communication unit 31.

Ahead-mounted display controller (HMD controller) 37 is connected to the HMD-side wireless communication unit 31, the image processing unit 32, the camera unit 33, and the display controller 35. The HMD-side wireless communication unit 31, the image processing unit 32, the display controller 35, and the HMD controller 37 configure a head-mounted display-side processor (HMD-side processor) 39.

The external apparatus 4 comprises an external wireless communication unit 41 that is connected to the ultrasound probe 2 and the HMD 3 by wireless communication, and an image processing unit 42 and an image synchronization unit 43 are connected to the external wireless communication unit 41. The image processing unit 42 is connected to the image synchronization unit 43. A display controller 44 and an external monitor 45 are sequentially connected to the image synchronization unit 43.

An external controller 46 is connected to the external wireless communication unit 41, the image processing unit 42, the image synchronization unit 43, and the display controller 44. An input device 47 is connected to the external controller 46. The external wireless communication unit 41, the image processing unit 42, the image synchronization unit 43, the display controller 44, and the external controller 46 configure an external apparatus-side processor 48.

The transducer array 21 of the ultrasound probe 2 has a plurality of transducers arranged in a one-dimensional or two-dimensional manner. Each transducer transmits an ultrasonic wave in response to a drive signal supplied from the transmission and reception circuit 22, receives an ultrasound echo from a subject, and outputs a reception signal based on the ultrasound echo. Each transducer is constituted by forming electrodes at both ends of a piezoelectric body made of, for example, piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

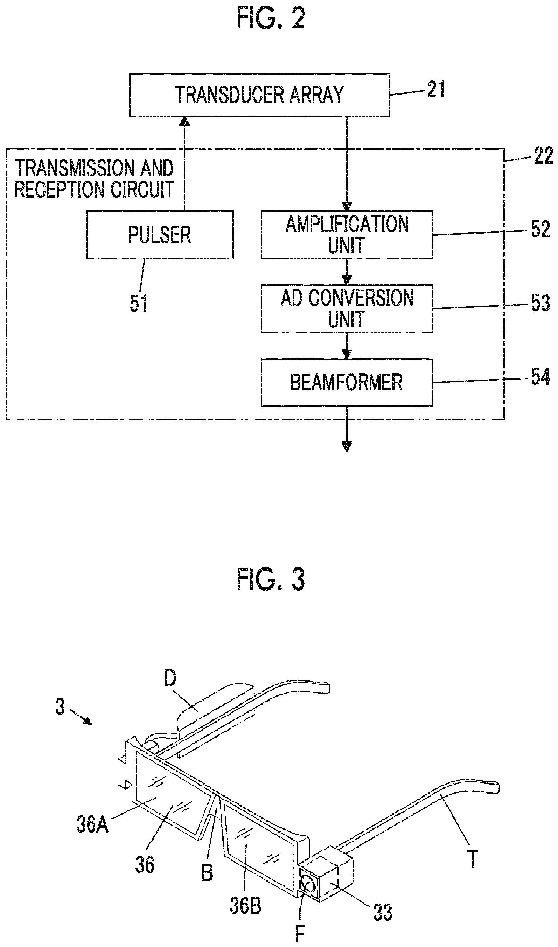

The transmission and reception circuit 22 transmits an ultrasonic wave from the transducer array 21 and generates a sound ray signal based on the reception signal acquired by the transducer array 21 under the control of the probe controller 26. As shown in Fig. 2, the transmission and reception circuit 22 has a pulser 51 that is connected to the transducer array 21, and an amplification unit 52, an analog-digital (AD) conversion unit 53, and a beamformer 54 connected in series from the transducer array 21.

The pulser 51 includes, for example, a plurality of pulse generators, and adjusts a delay amount of each drive signal based on a transmission delay pattern selected in response to a control signal from the probe controller 26 such that the ultrasonic waves transmitted from a plurality of transducers of the transducer array 21 form an ultrasonic beam, and supplies the drive signals to a plurality of transducers. In this way, in a case where a pulsed or continuous-wave voltage is applied to the electrodes of the transducers of the transducer array 21, the piezoelectric body expands and contracts to generate a pulsed or continuous-wave ultrasonic wave from each of the transducers. An ultrasonic beam is formed from a combined wave of the ultrasonic waves.

The transmitted ultrasonic beam is reflected by, for example, a target, such as a part of the subject, and propagates toward the transducer array 21 of the ultrasound probe 2. The ultrasound echo propagating toward the transducer array 21 is received by each transducer configuring the transducer array 21, and each transducer expands and contracts with reception of the propagating ultrasound echo to generate a reception signal as an electrical signal, and outputs the reception signal to the amplification unit 52.

The amplification unit 52 amplifies the signal input from each transducer constituting the transducer array 21 and transmits the amplified signal to the AD conversion unit 53. The AD conversion unit 53 converts the signal transmitted from the amplification unit 52 into digital reception data and transmits the reception data to the beamformer 54. The beamformer 54 executes so-called reception focus processing by giving a delay to each piece of reception data converted by the AD conversion unit 53 conforming to a sound speed or a distribution of a sound speed based on a reception delay pattern selected in response to a control signal from the probe controller 26 and performing addition. With the reception focus processing, each piece of reception data converted by the AD conversion unit 53 is phased and added, and a sound ray signal in which the focus of the ultrasound echo is narrowed is acquired.

The signal processing unit 23 generates reception data before imaging by executing signal processing on the sound ray signal generated by the beamformer 54 of the transmission and reception circuit 22. More specifically, the signal processing unit 23 performs correction of attenuation on the sound ray signal generated by the beamformer 54 of the transmission and reception circuit 22 due to a propagation distance depending on a depth of a position where the ultrasonic wave is reflected, and then, executes envelope detection processing to generate a signal representing tomographic image information regarding a tissue in the subject as reception data before imaging.

The probe-side wireless communication unit 24 includes an antenna that performs transmission and reception of radio waves, and modulates a carrier based on the reception data before imaging generated by the signal processing unit 23 to generate a transmission signal representing the reception data before imaging. The probe-side wireless communication unit 24 supplies the transmission signal generated in this manner to the antenna and transmits the radio waves from the antenna, thereby sequentially wirelessly transmitting the reception data before imaging to the HMD-side wireless communication unit 31 of the HMD 3 and the external wireless communication unit 41 of the external apparatus 4. As a modulation system of the carrier, for example, amplitude shift keying (ASK), phase shift keying (PSK), quadrature phase shift keying (QPSK), 16 quadrature amplitude modulation (16QAM), or the like is used.

The wireless communication among the probe-side wireless communication unit 24 of the ultrasound probe 2, the HMD-side wireless communication unit 31 of the HMD 3, and the external wireless communication unit 41 of the external apparatus 4 can be performed conforming to a communication standard regarding mobile communication, such as a 5th Generation mobile communication system (5G) or a 4th Generation mobile communication system (4G), or a communication standard regarding short-distance wireless communication, such as WiFi (Registered Trademark), Bluetooth (Registered Trademark), or an ultra wide band wireless system (UWB).

It is assumed that the ultrasound probe 2 and the HMD 3 are positioned close to each other, and thus, as the wireless communication between the ultrasound probe 2 and the HMD 3, any communication system of mobile communication or short-distance wireless communication may be employed.

It is assumed that the external apparatus 4 is positioned at a remote location with respect to the ultrasound probe 2 and the HMD 3, and thus, it is preferable that, as the wireless communication between the external apparatus 4 and the ultrasound probe 2 and the wireless communication between the external apparatus 4 and the HMD 3, mobile communication is performed. In particular, from a viewpoint of reducing a time lag in transmission of data between the external apparatus 4, and the ultrasound probe 2 and the HMD 3, it is preferable that, as the wireless communication between the external apparatus 4 and the ultrasound probe 2 and the wireless communication between the external apparatus 4 and the HMD 3, mobile communication conforming to 5G is performed.

The probe controller 26 performs control of each unit of the ultrasound probe 2 based on a control program and the like stored in advance.

Though not shown, a probe-side storage unit is connected to the probe controller 26. The probe-side storage unit stores the control program and the like of the ultrasound probe 2. As the probe-side storage unit, for example, a flash memory, a random access memory (RAM), a secure digital card (SD), or a solid state drive (SSD) can be used.

Though not shown, a battery is incorporated in the ultrasound probe 2, and power is supplied from the battery to each circuit of the ultrasound probe 2.

Although the probe-side processor 27 having the signal processing unit 23, the probe-side wireless communication unit 24, and the probe controller 26 is configured with a central processing unit (CPU) and a control program causing the CPU to execute various kinds of processing, the probe-side processor 27 may be configured using a field programmable gate array (FPGA), a digital signal processor (DSP), an application specific integrated circuit (ASIC), a graphics processing unit (GPU), or other integrated circuits (ICs) or may be configured by combining such ICs.

The signal processing unit 23, the probe-side wireless communication unit 24, and the probe controller 26 of the probe-side processor 27 may be configured to be partially or wholly integrated into one CPU or the like.

The HMD 3 is a display device that is mounted on a head of an operator and is viewed by the operator who mounts the HMD 3, and as shown in Fig. 3, has a shape of so-called spectacles. The HMD 3 comprises two monitors 36A and 36B as an HMD-side monitor 36. The two monitors 36A and 36B are connected to each other by a bridge portion B, and temple portions T are connected to end portions of the two monitors 36A and 36B, respectively. For example, the bridge portion B is placed and fixed on a nose of the operator and the two temple portions T are placed and fixed on both ears of the operator, whereby the HMD 3 is fixed to the head of the operator. In this case, the two monitors 36A and 36B face right and left eyes of the operator.

The camera unit 33 with an imaging lens F disposed on a front surface is attached to a connecting portion of the left monitor 36B and the temple portion T as viewed from the operator in a state in which the operator mounts the HMD 3. An accommodation portion D where various circuits and the like necessary for the operation of the HMD 3 is disposed in the temple portion T connected to the right monitor 36A.

Here, the HMD-side wireless communication unit 31 shown in Fig. 1 includes an antenna that performs transmission and reception of radio waves, and receives the transmission signal representing the reception data before imaging transmitted from the probe-side wireless communication unit 24 of the ultrasound probe 2, through the antenna and outputs the reception data before imaging by demodulating the received transmission signal under the control of the HMD controller 37. The HMD-side wireless communication unit 31 sends the reception data before imaging to the image processing unit 32.

The image processing unit 32 raster-converts the reception data before imaging sent from the HMD-side wireless communication unit 31 into an image signal conforming to a normal television signal scanning system and executes various kinds of necessary image processing, such as brightness correction, gradation correction, sharpness correction, an image size correction, refresh rate correction, scanning frequency correction, and color correction, conforming to a display format for the HMD-side monitor 36 on the converted image signal, thereby generating a brightness mode (B mode) image signal. The B mode image signal generated in this manner is simply referred to as an ultrasound image U. The image processing unit 32 sends the generated ultrasound image U to the display controller 35.

The HMD-side monitor 36 displays the ultrasound image U and the like under the control of the display controller 35, and has transmittance to secure the field of view of the operator in a state in which the operator mounts the HMD 3. For this reason, the operator can confirm the ultrasound image U and the like displayed on the HMD-side monitor 36 while confirming the field of view in front through the HMD-side monitor 36.

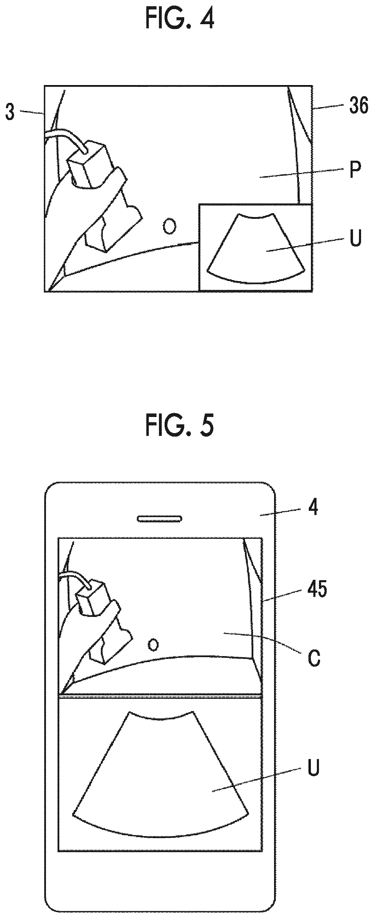

The display controller 35 executes predetermined processing on the ultrasound image U sent from the image processing unit 32 and displays the ultrasound image U on the HMD-side monitor 36 of the HMD 3 as shown in Fig. 4 under the control of the HMD controller 37. Fig. 4 shows an example where a subject P positioned in front of the operator is confirmed by the operator through the HMD-side monitor 36 and the ultrasound image U is displayed on the HMD-side monitor 36 to be superimposed on a part of the field of view in front of the operator.

The camera unit 33 acquires a view image C obtained by imaging a scanning point of the ultrasound probe 2 in the subject P. Te camera unit 33 incorporates the imaging lens F, an image sensor (not shown) that images the scanning point of the ultrasound probe 2 through the imaging lens F to acquire a view image signal as an analog signal, an analog signal processing circuit (not shown) that amplifies the view image signal acquired by the image sensor and converts the view image signal into a digital signal, and a digital signal processing circuit (not shown) that performs various kinds of correction, such as a gain, on the converted digital signal to generate the view image C. The analog signal processing circuit and the digital signal processing circuit may be incorporated in an HMD-side processor 39. The camera unit 33 sends the generated view image C to the HMD-side wireless communication unit 31. The HMD-side wireless communication unit 31 wirelessly transmits the view image C sent to the HMD-side wireless communication unit 31, to the external apparatus 4.

The HMD controller 37 performs control of each unit of the HMD 3 based on a control program and the like stored in advance.

Though not shown, an HMD-side storage unit is connected to the HMD controller 37. The HMD-side storage unit stores the control program and the like of the HMD 3. As the HMD-side storage unit, for example, a flash memory, a RAM, an SD card, or an SSD can be used.

Though not shown, a battery is incorporated in the HMD 3, and power is supplied from the battery to each circuit of the HMD 3.

Although the HMD-side processor 39 having the HMD-side wireless communication unit 31, the image processing unit 32, the display controller 35, and the HMD controller 37 is configured with a CPU and a control program causing the CPU to execute various kinds of processing, the HMD-side processor 39 may be configured using an FPGA, a DSP, an ASIC, a GPU, or other ICs or may be configured by combining such ICs.

The HMD-side wireless communication unit 31, the image processing unit 32, the display controller 35, and the HMD controller 37 of the HMD-side processor 39 may be configured to be partially or wholly integrated into one CPU or the like..

The external wireless communication unit 41 of the external apparatus 4 includes an antenna that performs transmission and reception of radio waves, and receives the transmission signal representing the reception data before imaging transmitted from the probe-side wireless communication unit 24 of the ultrasound probe 2 and a transmission signal representing the view image C transmitted from the HMD-side wireless communication unit 31 of the HMD 3 and outputs the reception data before imaging and the view image C by demodulating the received transmission signals under the control of the external controller 46. The external wireless communication unit 41 sends the reception data before imaging to the image processing unit 42 and sends the view image C to the image synchronization unit 43.

The image processing unit 42 raster-converts the reception data before imaging sent from the external wireless communication unit 41 into an image signal conforming to a normal television signal scanning system and executes various kinds of necessary image processing, such as brightness correction, gradation correction, sharpness correction, image size correction, refresh rate correction, scanning frequency correction, and color correction, conforming to a display format for the external monitor 45 on the converted image signal, thereby generating an ultrasound image U. The image processing unit 42 sends the generated ultrasound image U to the image synchronization unit 43.

The image synchronization unit 43 of the external apparatus 4 synchronizes the ultrasound image U sent from the image processing unit 42 and the view image C sent from the external wireless communication unit 41 with each other to generate a composite image M based on the ultrasound image U and the view image C synchronized with each other. Here, synchronizing the ultrasound image U and the view image C with each other refers to associating the ultrasound image U and the view image C captured at the same timing with each other. For example, in a case where a time stamp representing at time at which the ultrasound image U is generated is given to the ultrasound image U by the image processing unit 42 of the external apparatus 4, and a time stamp representing a time at which the view image C is generated is given to the view image C by the camera unit 33 of the HMD 3, the image synchronization unit 43 can synchronize the ultrasound image U and the view image C captured at the same timing with each other by regarding the time stamp of the ultrasound image U as representing a time at which the ultrasound image U is captured, regarding the time stamp of the view image C as representing a time at which the view image C is captured, and referring to the time stamps of the ultrasound image U and the view image C.

In associating the ultrasound image U and the view image C with each other, for example, the image synchronization unit 43 can refer to the time stamp of the ultrasound image U and the time stamp of the view image C, and in a case where a difference between the time at which the ultrasound image U is captured and the time at which the view image C is captured is within a given range, for example, within 0.1 seconds, can regard the ultrasound image U and the view image C as being captured at the same timing to perform association. Alternatively, for example, the image synchronization unit 34 may refer to the time stamp of the ultrasound image U and the time stamp of the view image C, may select the view image C captured at a time closest to the time at which the ultrasound image U to be associated is captured, and may associate the selected view image C and the ultrasound image U with each other. For example, the image synchronization unit 34 may select the ultrasound image U captured at a time closest to the time at which the view image C to be associated is captured, and may associate the selected ultrasound image U and the view image C with each other.

The image synchronization unit 43 sends the ultrasound image U and the view image C synchronized in this manner to the display controller 44.

The display controller 44 executes predetermined processing on the composite image M sent from the image synchronization unit 43 and displays the ultrasound image U and the view image C synchronized with each other together on the external monitor 45 of the external apparatus 4 as shown in Fig. 5 under the control of the external controller 46.

The external monitor 45 displays the ultrasound image U, the view image C, and the like under the control of the display controller 44, and includes, for example, a display device, such as a liquid crystal display (LCD) or an organic electroluminescence display (organic EL display).

The input device 47 of the external apparatus 4 is provided for the operator to perform an input operation, and includes a touch sensor disposed on the external monitor 45 in a superimposed manner.

The external controller 46 performs control of each unit of the external apparatus 4 based on a control program and the like stored in advance.

Though not shown, an external apparatus-side storage unit is connected to the external apparatus 4. The external apparatus-side storage unit stores the control program and the like of the external apparatus 4. As the external apparatus-side storage unit, for example, a flash memory, a RAM, an SD card, or an SSD can be used.

Though not shown, a battery is incorporated in the external apparatus 4, and power is supplied from the battery to each circuit of the external apparatus 4.

Although the external apparatus-side processor 48 having the external wireless communication unit 41, the image processing unit 42, the image synchronization unit 43, the display controller 44, and the external controller 46 is configured with a CPU and a control program causing the CPU to execute various kinds of processing, the external apparatus-side processor 48 may be constituted using an FPGA, a DSP, an ASIC, a GPU, or other ICs or may be constituted by combining such ICs.
The external wireless communication unit 41, the image processing unit 42, the image synchronization unit 43, the display controller 44, and the external controller 46 of the external apparatus-side processor 48 may be configured to be partially or wholly integrated into one CPU or the like.

Next, the operation of the ultrasound system 1 according to Embodiment 1 of the present invention will be described.

First, the ultrasound probe 2 is brought into contact with a body surface of the subject P by the operator, and ultrasonic beams are transmitted from a plurality of transducers of the transducer array 21 into the subject P in response to the drive signals from the pulser 51 of the transmission and reception circuit 22 under the control of the probe controller 26. An ultrasound echo based on the transmitted ultrasonic beam is received by each transducer, the reception signal as an analog signal is output to the amplification unit 52 and amplified, and is AD-converted by the AD conversion unit 53, and reception data is acquired. The beamformer 54 executes the reception focus processing on the reception data to generate a sound ray signal.

The signal processing unit 23 converts the generated sound ray signal into reception data before imaging that is a signal representing tomographic image information regarding a tissue in the subject P. In this case, the signal processing unit 23 performs correction of attenuation on the sound ray signal due to a propagation distance depending on a depth of a position where the ultrasonic wave is reflected, and then, executes envelope detection processing.

The probe-side wireless communication unit 24 wirelessly transmits the generated sound ray signal to the HMD 3 and the external apparatus 4.

The HMD-side wireless communication unit 31 of the HMD 3 receives the reception data before imaging wirelessly transmitted from the ultrasound probe 2 and sends the received reception data before imaging to the image processing unit 32. The image processing unit 32 raster-converts the reception data before imaging sent from the HMD-side wireless communication unit 31 into an image signal conforming to a normal television signal scanning system and executes various kinds of necessary image processing, such as brightness correction, gradation correction, sharpness correction, an image size correction, refresh rate correction, scanning frequency correction, and color correction, conforming to a display format for the HMD-side monitor 36 on the converted image signal, thereby generating the ultrasound image U. The ultrasound image U generated in this manner is sent to the display controller 35.

The display controller 35 executes predetermined processing on the ultrasound image U, then, sends the ultrasound image U to the HMD-side monitor 36, and displays the ultrasound image U on the HMD-side monitor 36 as shown in Fig. 4. The ultrasound image U is displayed on the HMD-side monitor 36 to be superimposed on a part of the field of view in front of the operator, and thus, the operator can simultaneously confirm the field of view in front and the ultrasound image U to easily correspond and recognize the scanning point of the ultrasound probe 2 in the subject P and the tissue in the subject P to be observed at the scanning point. An example of Fig. 4 shows a state in which the ultrasound probe 2 is in contact with an abdomen of the subject P as the field of view in front of the operator, and an internal tissue of the abdomen of the subject P is rendered in the ultrasound image U.

The camera unit 33 of the HMD 3 acquires the view image C obtained by imaging the scanning point of the ultrasound probe 2 in the subject P under the control of the HMD controller 37. For example, as shown in Fig. 4, the operator turns the field of view in front toward the subject P, whereby the view image C obtained by imaging the scanning point of the ultrasound probe 2 in the subject P is acquired by the camera unit 33. The view image C acquired in this manner is sent to the HMD-side wireless communication unit 31.

The HMD-side wireless communication unit 31 wirelessly transmits the view image C sent from the camera unit 33, to the external apparatus 4.

The external wireless communication unit 41 of the external apparatus 4 receives the reception data before imaging wirelessly transmitted from the ultrasound probe 2 and the view image C wirelessly transmitted from the HMD 3, sends the received reception data before imaging to the image processing unit 42, and sends the received view image C to the image synchronization unit 43.

The image processing unit 42 raster-converts the reception data before imaging sent from the external wireless communication unit 41 into an image signal conforming to a normal television signal scanning system and executes various kinds of necessary image processing, such as brightness correction, gradation correction, sharpness correction, image size correction, refresh rate correction, scanning frequency correction, and color correction, conforming to a display format for the external monitor 45 on the converted image signal, thereby generating an ultrasound image U. The image processing unit 42 sends the generated ultrasound image U to the image synchronization unit 43.

The image synchronization unit 43 synchronizes the ultrasound image U sent from the image processing unit 42 and the view image C sent from the external wireless communication unit 41 with each other to generate the composite image M in which the ultrasound image U and the view image C synchronized with each other are put together into one image. For example, in a case where a time stamp representing at time at which the ultrasound image U is generated is given to the ultrasound image U by the image processing unit 42 of the external apparatus 4, and a time stamp representing a time at which the view image C is generated is given to the view image C by the camera unit 33 of the HMD 3, the image synchronization unit 43 can synchronize the ultrasound image U and the view image C captured at the same timing with each other by regarding the time stamp of the ultrasound image U as representing a time at which the ultrasound image U is captured, regarding the time stamp of the view image C as representing a time at which the view image C is captured, and referring to the time stamps of the ultrasound image U and the view image C. In this case, the time in the HMD 3 and the time in the external apparatus 4 can be shared by each other.

Although the time in the HMD 3 and the time in the external apparatus 4 can be shared by each other, specifically, for example, the time can be shared with the HMD 3 or the external apparatus 4 as a reference. For example, in a case where any one of the HMD 3 or the external apparatus 4 is connected to the Internet, a time of an internal timepiece may be set using a communication protocol, such as Network Time Protocol (NTP) or Network Identity and Time Zone (NITZ).

The ultrasound image U and the view image C synchronized with each other by the image synchronization unit 43 are sent as the composite image M to the display controller 44. The display controller 44 executes predetermined processing on the composite image M, then, sends the composite image M to the external monitor 45, and displays the ultrasound image U and the view image C together on the external monitor 45 as shown in Fig. 5. An example of Fig. 5 shows that the external apparatus 4 is a mobile thin type computer, such as a so-called smartphone or a tablet, and the view image C and the ultrasound image U are displayed on the upper and lower sides of the external monitor 45 of the external apparatus 4, respectively. In this example, a state in which the ultrasound probe 2 is in contact with the abdomen of the subject P is rendered in the view image C, and the internal tissue of the abdomen of the subject P is rendered in the ultrasound image U.

Here, although the ultrasound image U is displayed on the HMD-side monitor 36 of the HMD 3 and the view image C and the ultrasound image U synchronized with each other are displayed on the external monitor 45 of the external apparatus 4, the same ultrasound image U is displayed on the HMD-side monitor 36 and the external monitor 45 substantially simultaneously. The view image corresponding to the field of view in front to be observed by the operator through the HMD-side monitor 36 is displayed on the external monitor 45 substantially in real time. For this reason, for example, even in a case where the external apparatus 4 is positioned at a remote location with respect to the HMD 3, the observer who observes the external monitor 45 can observe the ultrasound image U captured in a site of inspection where the subj ect P and the operator are positioned and the view image corresponding to the field of view in front of the operator substantially in real time.

Incidentally, in general, it is known that a given level or higher of skill is needed to accurately recognize the part in the subject P rendered in the ultrasound image by confirming the ultrasound image. Furthermore, it is known that the image quality of the ultrasound image generated in the ultrasound diagnostic apparatus significantly depends on the skill involving the hands of the operator.

For example, in a case where an ultrasound image is captured at a remote location other than a hospital, such as home care, the operator who operates the ultrasound probe to capture the ultrasound image may be different from the observer who observes the captured ultrasound image to perform diagnosis. In this case, since the operator normally needs to operate the ultrasound probe to capture an ultrasound image of an intended part in a subject P while confirming the obtained ultrasound image personally, in particular, in a case where the level of skill of the operator is low, the operator may hardly determine whether or not the intended part of the subject is accurately observed. The operator having a low level of skill may not operate the ultrasound probe using appropriate skill involving the hands, and an ultrasound image with low image quality is obtained.

The observer positioned at a remote location with respect to the subject P and the operator confirms the ultrasound image captured by the operator of the ultrasound diagnostic apparatus to perform diagnosis; however, since the observer cannot recognize a state in which the operator captures the ultrasound image, in particular, in a case where the ultrasound image is captured by the operator having a low level of skill, the observer may hardly accurately recognize whether or not the captured ultrasound image is captured by appropriate skill involving the hands.

With the ultrasound system 1 according to Embodiment 1 of the present invention, the same ultrasound image U is displayed on the HMD-side monitor 36 and the external monitor 45 substantially simultaneously, and the view image C corresponding to the field of view in front of the operator is displayed on the external monitor 45 substantially in real time. Thus, for example, even in a case where the external apparatus 4 is positioned at a remote location with respect to the HMD 3, the observer who observes the external monitor 45 can observe the view image C and the ultrasound image U captured in a site of inspection where the subject and the operator are positioned, substantially in real time. With this, for example, since the observer having a high level of skill can give advice to the operator in real time, even in a case where the level of skill of the operator positioned at a remote location with respect to the observer is low, an appropriate ultrasound image U is obtained, and it is possible to improve accuracy of ultrasound diagnosis.

With the ultrasound system 1 according to Embodiment 1 of the present invention, for example, the observer who is positioned at a remote location with respect to the operator and has a low level of skill can also be made to confirm the view image C representing a state in which the operator having a high level of skill operates the ultrasound probe 2 and the appropriate ultrasound image U corresponding to the view image C. In this way, the ultrasound system 1 according to Embodiment 1 of the present invention is considerably useful even from a viewpoint of training.

In the ultrasound system 1 according to Embodiment 1 of the present invention, since the ultrasound image U is displayed on the HMD-side monitor 36 of the HMD 3, the operator can confirm the ultrasound image U while confirming the field of view in front. In addition, since the view image C is captured by the camera unit 33 of the HMD 3, the operator does not need to use hands to capture the view image C.

For this reason, the operator can perform more various inspections, such as performing skill involving the hands to insert a so-called puncture needle into the subject P using the other hand while operating the ultrasound probe 2 using one hand, without separating the line of sight from the subj ect P.

Although an example where the time stamp is given to the generated ultrasound image U in each of the image processing unit 32 of the HMD 3 and the image processing unit 42 of the external apparatus 4 has been described, instead of the image processing unit 32 of the HMD 3 and the image processing unit 42 of the external apparatus 4 giving the time stamp to the ultrasound image U, the signal processing unit 23 of the ultrasound probe 2 may give a time stamp to the signal subjected to the envelope detection processing. In this case, for example, the time in the ultrasound probe 2 and the time in the HMD 3 are shared by each other, whereby it is possible to synchronize the ultrasound image U generated by the image processing unit 32 of the HMD 3 and the view image C generated by the camera unit 33 with each other based on the signal given the time stamp, and to synchronize the ultrasound image U generated by the image processing unit 42 of the external apparatus 4 and the view image C generated by the camera unit 33 with each other.

Here, the time in the ultrasound probe 2 and the time in the HMD 3 can be shared, for example, with the ultrasound probe 2 or the HMD 3 as a reference. For example, in a case where any one of the ultrasound probe 2 or the HMD 3 is connected to the Internet, the time of the internal timepiece may be set using a communication protocol, such as NTP or NITZ.

A method of synchronizing the ultrasound image U and the view image C with each other is not limited to the method using the time stamp described above. For example, as disclosed in JP2011-183056A, an imaging timing of the ultrasound image U by the ultrasound probe 2 and an imaging timing of the view image C by the camera unit 33 of the HMD 3 are synchronized with each other, and a time difference between the time at which the ultrasound image U is captured and the time at which the view image C is captured is within a given range, for example, within 0.1 seconds, the image synchronization unit 43 of the external apparatus 4 can regard that the ultrasound image U and the view image C are captured at the same timing, and can synchronize the ultrasound image U and the view image C with each other.

Although the image synchronization unit 43 of the external apparatus 4 generates the composite image M in which the ultrasound image U and the view image C synchronized with each other are put together into one image, and sends the generated composite image M to the display controller 44, instead of generating the composite image M, each of the ultrasound image U and the view image C synchronized with each other may be sent to the display controller 44. In this case, the display controller 44 executes predetermined processing on each of the ultrasound image U and the view image C sent from the image synchronization unit 43 and displays the ultrasound image U and the view image C synchronized with each other together on the external monitor 45, for example, as shown in Fig. 5. For this reason, the observer who views the external monitor 45 can observe the view image C and the ultrasound image U captured in a site of inspection where the subject P and the operator are positioned, substantially in real time.

Though not shown, for example, the HMD 3 may comprise an eye camera unit that images eyes of the operator, and an eye movement detection unit that analyzes an eye image representing the eyes of the operator captured by the eye camera unit to detect a wink, the line of sight, or the like of the operator. The detected wink and movement of the light of sight of the operator can be used, for example, as an input operation of the operator. For example, the number of winks, the position of the light of sight, or the like of the operator detected by the eye movement detection unit can be used as a trigger or the like of imaging start and imaging stop of the view image C by the camera unit 33. In this way, the wink, the movement of the light of sight, or the like of the operator is used as an input operation, whereby it is possible to allow the operator to perform an input operation without using the hands even in a case where both hands are used during inspection of the subject P.

Although the ultrasound probe 2 and the HMD 3 are connected to each other by wireless communication, for example, the ultrasound probe 2 and the HMD 3 may be connected to each other by wired communication, instead of being connected by wireless communication.

In Fig. 4, although an example where the external apparatus 4 is a mobile thin type computer, such as a so-called smartphone or a tablet, has been illustrated, the external apparatus 4 is not limited thereto. For example, as the external apparatus 4, a so-called notebook type personal computer, a stationary type personal computer, or the like may be used.

As shown in Fig. 2, although the transmission and reception circuit 22 has the beamformer 54 together with the amplification unit 52 and the AD conversion unit 53, the beamformer 54 may be disposed between the transmission and reception circuit 22 and the signal processing unit 23, not inside the transmission and reception circuit 22. In this case, the probe-side processor 27 may configure the beamformer 54.

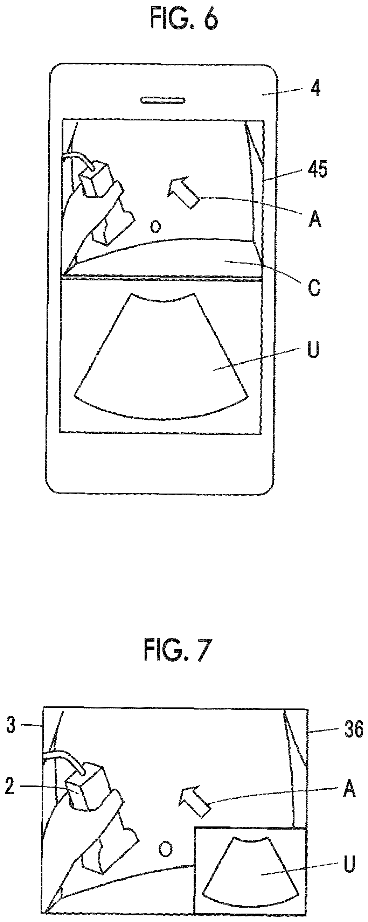

For example, as shown in Figs. 6 and 7, external input information, such as a cursor A that is movable by an input operation of the observer through the input device 47 of the external apparatus 4, can be displayed on the external monitor 45 of the external apparatus 4 and the HMD-side monitor 36 of the HMD 3 simultaneously. In this case, for example, in a case where the cursor A displayed on the external monitor 45 of the external apparatus 4 is moved by an input operation of the observer through the input device 47 of the external apparatus 4, the cursor A displayed on the terminal monitor 36 of the mobile information terminals is moved similarly. With this, for example, it is possible to perform more detailed information sharing between the operator of the ultrasound probe 2 and the HMD 3 and the observer positioned close to the external apparatus 4. For example, the observer having a high level of skill who observes the ultrasound image U and the view image C on the external monitor 45 of the external apparatus 4 can easily support inspection that is performed by the operator, such as indicating a position where the ultrasound probe 2 is to be scanned, to the operator having a low level of skill of the ultrasound probe 2 and the HMD 3 using the cursor A.

For example, in a case where an input operation can be performed through the HMD 3 by the movement of the light of sight or the like of the operator, the cursor A that is movable by an input operation of the operator may displayed on the HMD-side monitor 36 of the HMD 3 and the external monitor 45 of the external apparatus 4 simultaneously. In this case, for example, the operator having a high level of skill can perform training on ultrasound diagnosis for the observer having a low level of skill positioned close to the external apparatus 4 more easily and in more detail.

The shape of the cursor A is not limited to an arrow shape, and can have any shape, such as a circular shape or a polygonal shape.

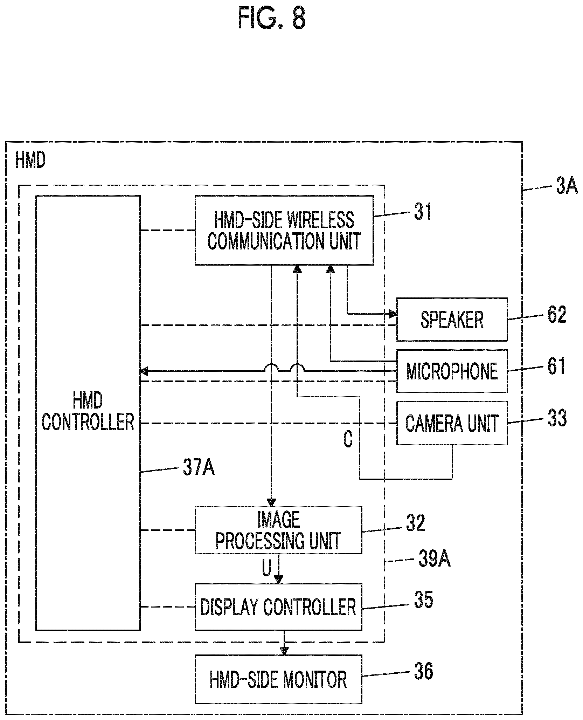

For example, wireless communication of voice data may be performed between the HMD 3 and the external apparatus 4 in two directions. Fig. 8 shows the configuration of an HMD 3A in a modification example of Embodiment 1 of the present invention. The HMD 3A is further provided with a microphone 61 and a speaker 62, comprises an HMD controller 37A instead of the HMD controller 37, and comprises an HMD-side processor 39A instead of the HMD-side processor 39, compared to the HMD 3 shown in Fig. 1. In the HMD 3A, the microphone 61 and the speaker 62 are connected to the HMD-side wireless communication unit 31.

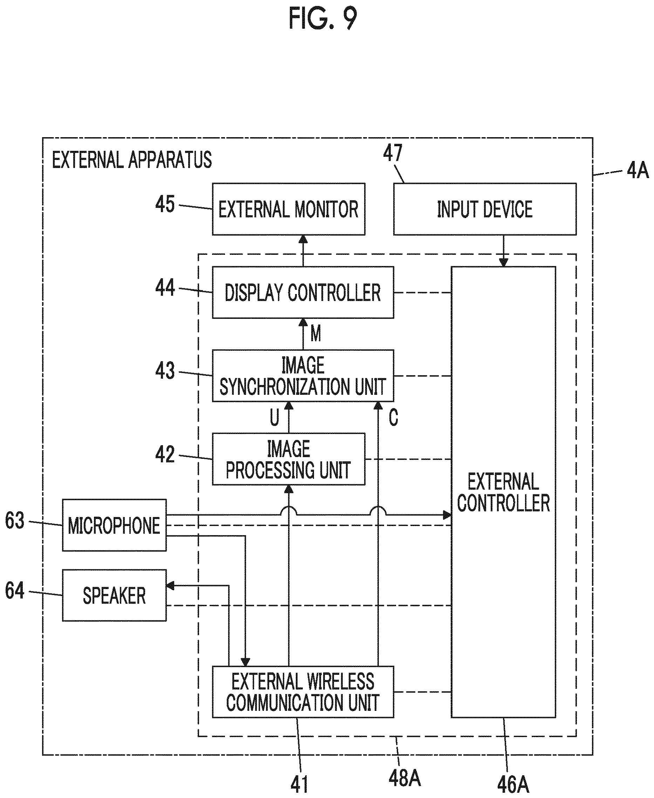

Fig. 9 shows the configuration of an external apparatus 4A in the modification example of Embodiment 1 of the present invention. The external apparatus 4A is further provided with a microphone 63 and a speaker 64, comprises an external controller 46A instead of the external controller 46, and comprises an external apparatus-side processor 48A instead of the external apparatus-side processor 48, compared to the external apparatus 4 shown in Fig. 1. In the external apparatus 4A, the microphone 63 and the speaker 64 are connected to the external wireless communication unit 41.

In the modification example of Embodiment 1 of the present invention, voice data is transmitted and received between the HMD 3A and the external apparatus 4A in two directions. For example, in a case where the operator of the ultrasound probe 2 and the HMD 3A utters voice toward the HMD 3A, the uttered voice is input to the microphone 61 of the HMD 3A, and voice data is generated by the microphone 61. The generated voice data is wirelessly transmitted from the HMD-side wireless communication unit 31 to the external apparatus 4A. The external wireless communication unit 41 of the external apparatus 4A receives the voice data wirelessly transmitted from the HMD 3A and sends the received voice data to the speaker 64. The speaker 64 reproduces the voice uttered by the operator of the ultrasound probe 2 and the HMD 3A based on the voice data received from the external wireless communication unit 41.

For example, the observer who observes the ultrasound image U and the view image C on the external monitor 45 of the external apparatus 4A utters voice toward the external apparatus 4A, the uttered voice is input to the microphone 63 of the external apparatus 4A, and voice data is generated by the microphone 63. The generated voice data is wirelessly transmitted from the external wireless communication unit 41 to the HMD 3A. The HMD-side wireless communication unit 31 of the HMD 3A receives the voice data wirelessly transmitted from the external apparatus 4A and sends the received voice data to the speaker 62. The speaker 62 reproduces the voice uttered by the observer positioned close to the external apparatus 4 based on the voice data received from the HMD-side wireless communication unit 31.

In this manner, the voice data is transmitted and received between the HMD 3A and the external apparatus 4A in two directions, whereby it is possible to perform more detailed information sharing between the operator of the ultrasound probe 2 and the HMD 3A and the observer positioned close to the external apparatus 4A. For example, the observer having a high level of skill who observes the ultrasound image U and the view image C on the external monitor 45 of the external apparatus 4A can give advice to the operator having a low level of skill of the ultrasound probe 2 and the HMD 3A more easily and in more detail. For example, the operator having a high level of skill can perform training on ultrasound diagnosis for the observer having a low level of skill positioned close to the external apparatus 4A more easily and in more detail.

The voice of the operator input to the microphone 61 of the HMD 3A may be used as an input operation of the operator. For example, the HMD controller 37A can acquire instruction information by analyzing the voice data generated based on the voice of the operator by the microphone 61 and can perform control of each unit of the HMD 3A, such as imaging start and imaging stop of the view image C by the camera unit 33, conforming to the acquired instruction information. Alternatively, the control of the ultrasound probe 2 may be performed based on the voice data analyzed by the HMD controller 37A. In this case, for example, the voice data analyzed by the HMD controller 37A is wirelessly transmitted as input information from the operator from the HMD-side wireless communication unit 31 to the ultrasound probe 2, and is input to the probe controller 26 by way of the probe-side wireless communication unit 24. The probe controller 26 can perform control of each unit of the ultrasound probe 2, such as transmission start and transmission stop of ultrasonic waves by the transducer array 21.

Alternatively, the voice of the observer input to the microphone 63 of the external apparatus 4A may be used as an input operation of the observer. For example, the external controller 46A may acquire instruction information by analyzing the voice data generated based on the voice of the observer by the microphone 63 and may perform control of each unit of the head-mounted display 3A, such as imaging start and imaging stop of the view image C by the camera unit 33 of the head-mounted display 3A, and control of each unit of the ultrasound probe 2, such as transmission start and transmission stop of ultrasonic waves by the transducer array 21 of the ultrasound probe 2.

### Embodiment 2

In Embodiment 1, although the reception data before imaging obtained by performing the envelope detection processing on the sound ray signal is generated in the ultrasound probe 2, and the generated reception data before imaging is wirelessly transmitted to the HMD 3 and the external apparatus 4, the ultrasound image U may be generated in the ultrasound probe 2, and the generated ultrasound image U may be wirelessly transmitted to the HMD 3 and the external apparatus 4.

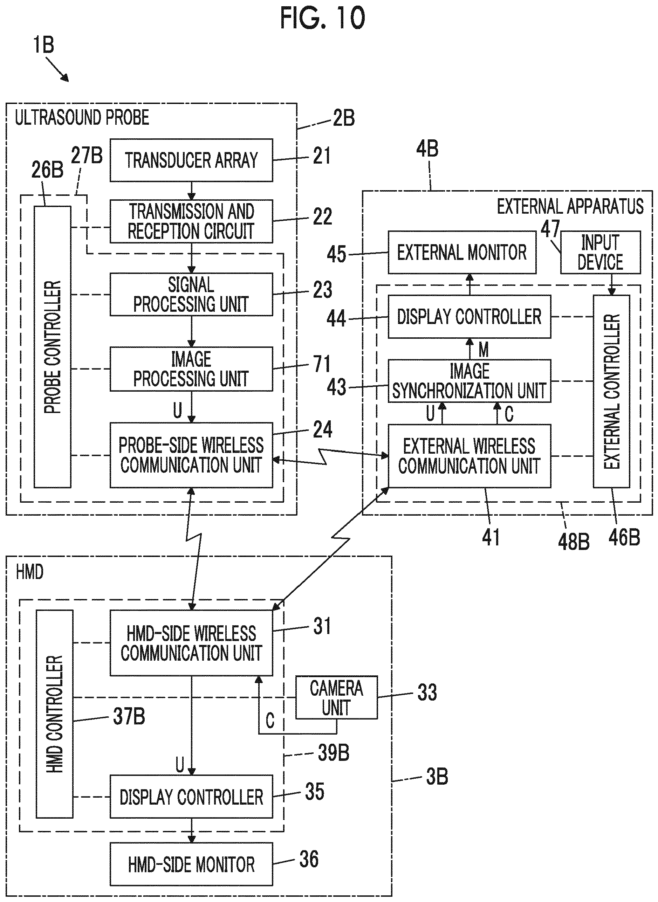

Fig. 10 shows the configuration of an ultrasound system 1B according to Embodiment 2 of the present invention. The ultrasound system 1B comprises an ultrasound probe 2B instead of the ultrasound probe 2, comprises an HMD 3B instead of the HMD 3, and comprises an external apparatus 4B instead of the external apparatus 4, compared to the ultrasound system 1 of Embodiment 1 shown in Fig. 1.

The ultrasound probe 2B is further provided with an image processing unit 71, comprises a probe controller 26B instead of the probe controller 26, and a probe-side processor 27B instead of the probe-side processor 27, compared to the ultrasound probe 2 in Embodiment 1. In the ultrasound probe 2B, the image processing unit 71 is connected to the signal processing unit 23. The probe-side wireless communication unit 24 and the probe controller 26B are connected to the image processing unit 71. Though not shown, the signal processing unit 23 and the image processing unit 71 configure an ultrasound image generation unit.

The HMD 3B is not provided with the image processing unit 32, comprises an HMD controller 37B instead of the HMD controller 37, and comprises an HMD-side processor 39B instead of the HMD-side processor 39, compared to the HMD 3 in Embodiment 1. In the HMD 3B, the display controller 35 and the camera unit 33 are connected to the HMD-side wireless communication unit 31.

The external apparatus 4B is not provided with the image processing unit 42, comprises an external controller 46B instead of the external controller 46, and comprises an external apparatus-side processor 48B instead of the external apparatus-side processor 48, compared to the external apparatus 4 in Embodiment 1.

The image processing unit 71 of the ultrasound probe 2B raster-converts the signal subjected to the envelope detection processing by the signal processing unit 23 into an image signal conforming to a normal television signal scanning system and executes various kinds of necessary image processing, such as brightness correction, gradation correction, sharpness correction, an image size correction, refresh rate correction, scanning frequency correction, and color correction, on the converted image signal, thereby generating an ultrasound image U conforming to a display format for the HMD-side monitor 36 of the HMD 3B and an ultrasound image U conforming to a display format for the external monitor 45 of the external apparatus 4B. The image processing unit 71 wirelessly transmits the ultrasound image U conforming to the display format for the HMD-side monitor 36 of the HMD 3B from the probe-side wireless communication unit 24 to the HMD 3B and wirelessly transmits the ultrasound image U conforming to the display format for the external monitor 45 of the external apparatus 4B from the probe-side wireless communication unit 24 to the external apparatus 4B.

The HMD-side wireless communication unit 31 of the HMD 3B receives the ultrasound image U wirelessly transmitted from the ultrasound probe 2B and sends the received ultrasound image U to the display controller 35.

The display controller 35 executes predetermined processing on the ultrasound image U sent from the HMD-side wireless communication unit 31, then, sends the ultrasound image U to the HMD-side monitor 36, and displays the ultrasound image U on the HMD-side monitor 36 as shown in Fig. 4.

The external wireless communication unit 41 of the external apparatus 4B receives the ultrasound image U wirelessly transmitted from the ultrasound probe 2B and the view image C wirelessly transmitted from the HMD 3B and sends the received ultrasound image U and view image C to the image synchronization unit 43.

The image synchronization unit 43 synchronizes the ultrasound image U and the view image C sent from the external wireless communication unit 41 with each other and generates a composite image M based on the ultrasound image U and the view image C synchronized with each other.

The display controller 44 executes predetermined processing on the composite image M generated by the image synchronization unit 43, then, sends the composite image M to the external monitor 45, and displays the ultrasound image U and the view image C synchronized with each other together on the external monitor 45 as shown in Fig. 5.

As described above, with the ultrasound system 1B according to Embodiment 2 of the present invention, even in a case where the ultrasound probe 2B comprises the image processing unit 71, similarly to the ultrasound system 1 of Embodiment 1 in which the HMD 3 comprises the image processing unit 32 and the external apparatus 4 comprises the image processing unit 42, the same ultrasound image U is displayed on the HMD-side monitor 36 and the external monitor 45 substantially simultaneously, and the ultrasound probe 2B and the view image C corresponding to the field of view in front of the operator of the HMD 3B are displayed on the external monitor 45 substantially in real time. For this reason, for example, since the observer who observes the view image C and the ultrasound image U with the external apparatus 4B disposed at a remote location can give advice to the operator of the ultrasound probe 2B and the HMD 3B, an appropriate ultrasound image U is obtained, and it is possible to improve accuracy of ultrasound diagnosis.

In the ultrasound system 1 of Embodiment 1 shown in Fig. 1, the HMD 3 comprises the image processing unit 32 and the external apparatus 4 comprises the image processing unit 42. In contrast, in the ultrasound system 1B according to Embodiment 2, the ultrasound probe 2B comprises the image processing unit 71. Thus, the HMD 3B and the external apparatus 4B do not need to have the image processing units 32 and 42, respectively, and the internal configurations of the HMD 3B and the external apparatus 4B are simplified compared to the internal configurations of the HMD 3 and the external apparatus 4 in the ultrasound system 1 of Embodiment 1. For this reason, with the ultrasound system 1B according to Embodiment 2, it is possible to reduce power consumption, calculation load, and the like of the HMD 3B and the external apparatus 4B compared to the ultrasound system 1 of Embodiment 1.

### Embodiment 3

In Embodiment 1, although the ultrasound image U and the view image C are synchronized in the external apparatus 4, for example, the ultrasound image U and the view image C may be synchronized in the HMD 3.

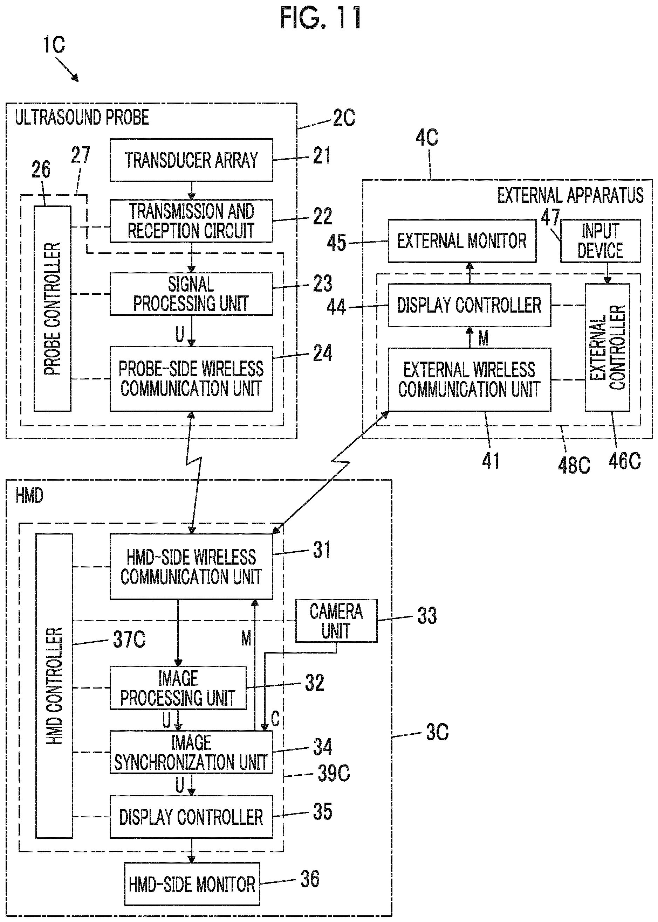

Fig. 11 shows the configuration of an ultrasound system 1C according to Embodiment 3 of the present invention. The ultrasound system 1C comprises an ultrasound probe 2C having the same internal configuration as the ultrasound probe 2, comprises an HMD 3C instead of the HMD 3, and comprises an external apparatus 4C instead of the external apparatus 4, compared to the ultrasound system 1 of Embodiment 1 shown in Fig. 1. The ultrasound probe 2C is connected only to the HMD 3C by wireless communication, and the external apparatus 4C is connected only to the HMD 3C by wireless communication.

The HMD 3C is further provided with an image synchronization unit 34, comprises an HMD controller 37C instead of the HMD controller 37, and comprises an HMD-side processor 39C instead of the HMD-side processor 39, compared to the HMD 3 in Embodiment 1. In the HMD 3C, the image synchronization unit 34 is connected to the HMD-side wireless communication unit 31, the image processing unit 32, and the camera unit 33. The display controller 35 is connected to the image synchronization unit 34.

The external apparatus 4C is not provided with the image processing unit 42 and the image synchronization unit 43, comprises an external controller 46C instead of the external controller 46, and comprises an external apparatus-side processor 48C instead of the external apparatus-side processor 48, compared to the external apparatus 4 in Embodiment 1. In the external apparatus 4C, the display controller 44 is connected to the external wireless communication unit 41.

The probe-side wireless communication unit 24 of the ultrasound probe 2C wirelessly transmits the reception data before imaging subjected to the envelope detection processing by the signal processing unit 23 only to the HMD 3C.

The HMD-side wireless communication unit 31 of the HMD 3C receives the reception data before imaging wirelessly transmitted from the ultrasound probe 2C and sends the received reception data before imaging to the image processing unit 32.

The image processing unit 32 raster-converts the reception data before imaging sent from the HMD-side wireless communication unit 31 into an image signal conforming to a normal television signal scanning system and executes various kinds of necessary image processing, such as brightness correction, gradation correction, sharpness correction, an image size correction, refresh rate correction, scanning frequency correction, and color correction, on the converted image signal, thereby generating an ultrasound image U conforming to a display format for the HMD-side monitor 36 of the HMD 3C and an ultrasound image U conforming to a display format for the external monitor 45 of the external apparatus 4C. The image processing unit 32 sends the ultrasound image U conforming to the display format for the HMD-side monitor 36 of the HMD 3C to the display controller 35 by way of the image synchronization unit 34 and sends the ultrasound image U conforming to the display format for the external monitor 45 of the external apparatus 4C to the image synchronization unit 34.

The display controller 35 executes predetermined processing on the ultrasound image U conforming to the display format for the HMD-side monitor 36 sent from the image processing unit 32 by way of the image synchronization unit 34, and then, displays the ultrasound image U on the HMD-side monitor 36 as shown in Fig. 4.

The camera unit 33 acquires a view image C obtained by imaging a scanning point of the ultrasound probe 2C in the subject P and sends the acquired view image C to the image synchronization unit 34.

The image synchronization unit 34 synchronizes the ultrasound image U sent from the image processing unit 32 and the view image C sent from the camera unit 33 with each other and generates a composite image M based on the ultrasound image U and the view image C synchronized with each other.

The image synchronization unit 34 sends the composite image M generated based on the ultrasound image U conforming to the display format for the external monitor 45 of the external apparatus 4C and the view image C to the HMD-side wireless communication unit 31.

The HMD-side wireless communication unit 31 wirelessly transmits the composite image M sent from the image synchronization unit 34, to the external apparatus 4C.

The external wireless communication unit 41 of the external apparatus 4C receives the composite image M wirelessly transmitted from the HMD 3C and sends the received composite image M to the display controller 44. The display controller 44 executes predetermined processing on the composite image M sent from the external wireless communication unit 41, then, sends the composite image M to the external monitor 45, and displays the ultrasound image U and the view image C synchronized with each other together on the external monitor 45 as shown in Fig. 5.

From the above description, with the ultrasound system 1C according to Embodiment 3 of the present invention, even in a case where only the HMD 3C comprises the image processing unit 32 and the image synchronization unit 34, similarly to the ultrasound system 1 of Embodiment 1 in which the HMD 3 comprises the image processing unit 32 and the external apparatus 4 comprises the image processing unit 42 and the image synchronization unit 43, the same ultrasound image U is displayed on the HMD-side monitor 36 and the external monitor 45 substantially simultaneously, and the ultrasound probe 2C and the view image C corresponding to the field of view in front of the operator of the HMD 3C are displayed on the external monitor 45 substantially in real time. For this reason, for example, since the observer who observes the view image C and the ultrasound image U with the external apparatus 4C disposed at a remote location can give advice to the operator of the ultrasound probe 2C and the HMD 3C, an appropriate ultrasound image U is obtained, and it is possible to improve accuracy of ultrasound diagnosis.

In the ultrasound system 1 of Embodiment 1 shown in Fig. 1, although the external apparatus 4 comprises the image processing unit 42 and the image synchronization unit 43. In contrast, in the ultrasound system 1C according to Embodiment 3, the composite image M generated based on the ultrasound image U and the view image C is wirelessly transmitted from the HMD 3C to the external apparatus 4C. Thus, the external apparatus 4C does not need to have the image processing unit 42 and the image synchronization unit 43, and the internal configuration of the external apparatus 4C is simplified compared to the internal configuration of the external apparatus 4 in Embodiment 1. For this reason, with the ultrasound system 1C according to Embodiment 3, it is possible to reduce power consumption, calculation load, and the like of the external apparatus 4C.

### Embodiment 4

In Embodiment 3, although the reception data before imaging subjected to the envelope detection processing by the signal processing unit 23 of the ultrasound probe 2 is wirelessly transmitted to the HMD 3 and the external apparatus 4, the ultrasound image U may be generated in the ultrasound probe 2.

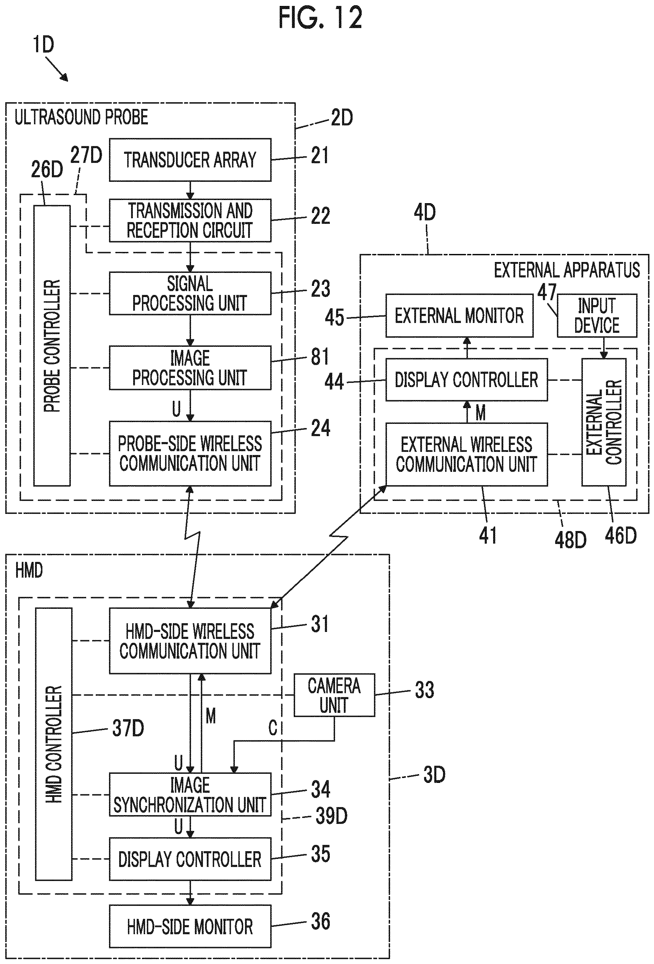

Fig. 12 shows the configuration of an ultrasound system 1D according to Embodiment 4 of the present invention. The ultrasound system 1D comprises an ultrasound probe 2D instead of the ultrasound probe 2C, comprises an HMD 3D instead of the HMD 3C, and comprises an external apparatus 4D instead of the external apparatus 4C, compared to the ultrasound system 1C of Embodiment 3 shown in Fig. 11. The ultrasound probe 2D is connected only to the HMD 3D by wireless communication, and the external apparatus 4D is connected only to the HMD 3D by wireless communication.

The ultrasound probe 2D is further provided with an image processing unit 81, comprises a probe controller 26D instead of the probe controller 26, and comprises a probe-side processor 27D instead of the probe-side processor 27, compared to the ultrasound probe 2C in Embodiment 3. In the ultrasound probe 2D, the image processing unit 81 is connected to the signal processing unit 23, and the probe-side wireless communication unit 24 and the probe controller 26D are connected to the image processing unit 81. Though not shown, the signal processing unit 23 and the image processing unit 81 configure an ultrasound image generation unit.

The HMD 3D is not provided with the image processing unit 32, comprises an HMD controller 37D instead of the HMD controller 37C, and comprises an HMD-side processor 39D instead of the HMD-side processor 39C, compared to the HMD 3C in Embodiment 3. In the HMD 3D, the image synchronization unit 34 is connected to the HMD-side wireless communication unit 31. The camera unit 33 is connected to the image synchronization unit 34.

The external apparatus 4D comprises an external controller 46D instead of the external controller 46C and comprise an external apparatus-side processor 48D instead of the external apparatus-side processor 48C, compared to the external apparatus 4C in Embodiment 3.

The image processing unit 81 of the ultrasound probe 2D raster-converts the signal subjected to the envelop detection processing by the signal processing unit 23 into an image signal conforming to a normal television signal scanning system and executes various kinds of necessary image processing, such as brightness correction, gradation correction, sharpness correction, an image size correction, refresh rate correction, scanning frequency correction, and color correction, on the converted image signal, thereby generating an ultrasound image U conforming to a display format for the HMD-side monitor 36 of the HMD 3D and an ultrasound image U conforming to a display format for the external monitor 45 of the external apparatus 4D. The image processing unit 81 sends the generated ultrasound images U to the probe-side wireless communication unit 24.

The probe-side wireless communication unit 24 wirelessly transmits the ultrasound image U sent from the image processing unit 81 to the HMD 3D.

The HMD-side wireless communication unit 31 receives the ultrasound image U wirelessly transmitted from the ultrasound probe 2D. The HMD-side wireless communication unit 31 sends the ultrasound image U conforming to the display format for the HMD-side monitor 36 to the display controller 35 by way of the image synchronization unit 34 and sends the ultrasound image U conforming to the display format for the external monitor 45 to the image synchronization unit 34.

The display controller 35 executes predetermined processing on the ultrasound image U sent from the HMD-side wireless communication unit 31 by way of the image synchronization unit 34 and displays the ultrasound image U on the HMD-side monitor 36 as shown in Fig. 4.

The camera unit 33 acquires a view image C obtained by imaging a scanning point of the ultrasound probe 2D in the subject P and sends the acquired view image C to the image synchronization unit 34.

The image synchronization unit 34 synchronizes the ultrasound image U sent from the HMD-side wireless communication unit 31 and the view image C sent from the camera unit 33 with each other and generates a composite image M based on the ultrasound image U and the view image C synchronized with each other.

The image synchronization unit 34 sends the composite image M generated based on the ultrasound image U conforming to the display format for the external monitor 45 and the view image C synchronized with each other to the HMD-side wireless communication unit 31.

The HMD-side wireless communication unit 31 wirelessly transmits the composite image M sent from the image synchronization unit 34 to the external apparatus 4D.

The external wireless communication unit 41 of the external apparatus 4D receives the composite image M wirelessly transmitted from the HMD 3D and sends the received composite image M to the display controller 44.

The display controller 44 executes predetermined processing on the composite image M sent from the external wireless communication unit 41, then, sends the composite image M to the external monitor 45, and displays the ultrasound image U and the view image C synchronized with each other together on the external monitor 45 as shown in Fig. 5.

From the above description, with the ultrasound system 1D according to Embodiment 4, even in a case where only the ultrasound probe 2D comprises the image processing unit 81 and only the HMD 3D comprises the image synchronization unit 34, similarly to the ultrasound system 1 of Embodiment 1 in which the HMD 3 comprises the image processing unit 32 and the external apparatus 4 comprises the image processing unit 42 and the image synchronization unit 43, the same ultrasound image U is displayed on the HMD-side monitor 36 and the external monitor 45 substantially simultaneously, and the ultrasound probe 2C and the view image C corresponding to the field of view in front of the operator of the HMD 3C are displayed on the external monitor 45 substantially in real time. For this reason, for example, since the observer who observes the view image C and the ultrasound image U with the external apparatus 4D disposed at a remote location can give advice to the operator of the ultrasound probe 2D and the HMD 3D, an appropriate ultrasound image U is obtained, and it is possible to improve accuracy of ultrasound diagnosis.

### Embodiment 5

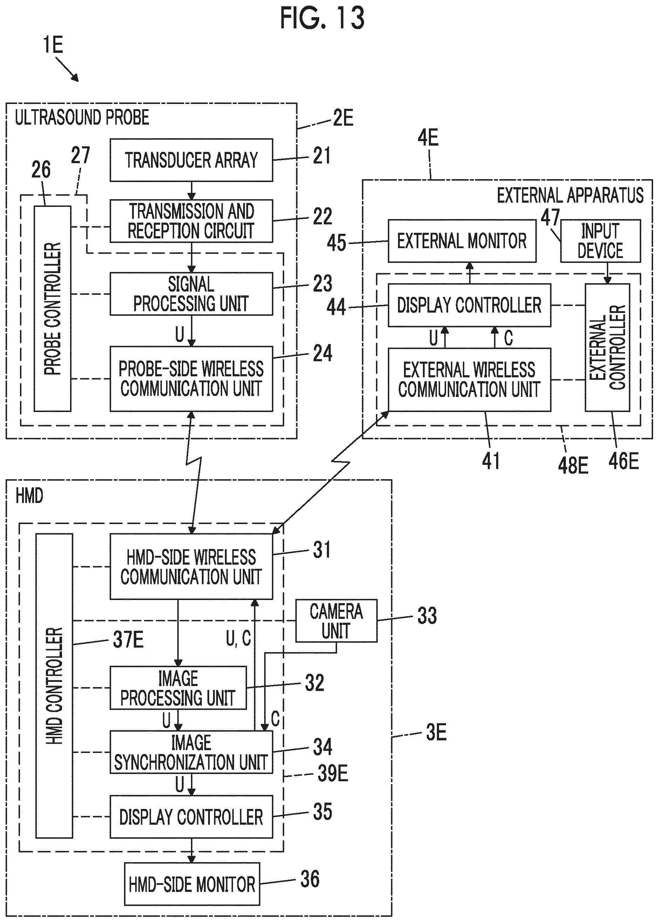

In Embodiment 3, since the external apparatus 4C receives the composite image M from the HMD 3C and displays the received composite image M on the external monitor 45, the disposition and the size of the ultrasound image U and the view image C displayed on the external monitor 45 cannot be freely changed on the external apparatus 4C side. In contrast, with an ultrasound system 1E of Embodiment 5 shown in Fig. 13, the disposition and the size of the ultrasound image U and the view image C displayed on the external monitor 45 can be arbitrarily changed on the external apparatus 4E side.

Fig. 13 shows the configuration of an ultrasound system 1E according to Embodiment 5 of the present invention. The ultrasound system 1E comprises an ultrasound probe 2E having the same internal configuration as the ultrasound probe 2C, comprises an HMD 3E instead of the HMD 3C, and comprises an external apparatus 4E instead of the external apparatus 4C, compared to the ultrasound system 1C of Embodiment 3. The ultrasound probe 2E is connected only to the HMD 3E by wireless communication, and the external apparatus 4E is connected only to the HMD 3E by wireless communication.

The HMD 3E comprises an HMD controller 37E instead of the HMD controller 37, and comprises an HMD-side processor 39E instead of the HMD-side processor 39, compared to the HMD 3C in Embodiment 3. In the HMD 3E, the image synchronization unit 34 is connected to the HMD-side wireless communication unit 31, the image processing unit 32, and the camera unit 33, and the display controller 35 is connected to the image synchronization unit 34.

The external apparatus 4E comprises an external controller 46E instead of the external controller 46 and comprises an external apparatus-side processor 48E instead of the external apparatus-side processor 48, compared to the external apparatus 4C in Embodiment 3.

The probe-side wireless communication unit 24 of the ultrasound probe 2E wirelessly transmit the reception data before imaging subjected to the envelope detection processing by the signal processing unit 23 to the HMD 3E.

The HMD-side wireless communication unit 31 of the HMD 3E receives the reception data before imaging wirelessly transmitted from the ultrasound probe 2E and sends the received reception data before imaging to the image processing unit 32.

The image processing unit 32 generates an ultrasound image U conforming to a display format for the HMD-side monitor 36 of the HMD 3E and an ultrasound image U conforming to a display format for the external monitor 45 of the external apparatus 4E based on the reception data before imaging sent from the HMD-side wireless communication unit 31. The image processing unit 32 sends the ultrasound image U conforming to the display format for the HMD-side monitor 36 to the display controller 35 by way of the image synchronization unit 34 and sends the ultrasound image U conforming to the display format for the HMD-side monitor 36 to the image synchronization unit 34.

The display controller 35 executes predetermined processing on the ultrasound image U sent from the image processing unit 32 by way of the image synchronization unit 34, and then, displays the ultrasound image U on the HMD-side monitor 36 as shown in Fig. 4.

The camera unit 33 acquires a view image C obtained by imaging a scanning point of the ultrasound probe 2E in the subject P and sends the acquired view image C to the image synchronization unit 34.

The image synchronization unit 34 synchronizes the ultrasound image U sent from the HMD-side wireless communication unit 31 and the view image C sent from the camera unit 33 with each other.

The image synchronization unit 34 sends each of the ultrasound image U and the view image C to the HMD-side wireless communication unit 31, instead of generating one composite image M based on the ultrasound image U conforming to the display format for the external monitor 45 and the view image C synchronized with each other.

The HMD-side wireless communication unit 31 wirelessly transmits the ultrasound image U and the view image C sent from the image synchronization unit 34 to the external apparatus 4E.

The external wireless communication unit 41 of the external apparatus 4E receives the ultrasound image U and the view image C wirelessly transmitted from the HMD 3E and sends each of the received ultrasound image U and view image C to the display controller 44.

The display controller 44 executes predetermined processing on the ultrasound image U and the view image C sent from the external wireless communication unit 41 and displays the ultrasound image U and the view image C synchronized with each other together on the external monitor 45.

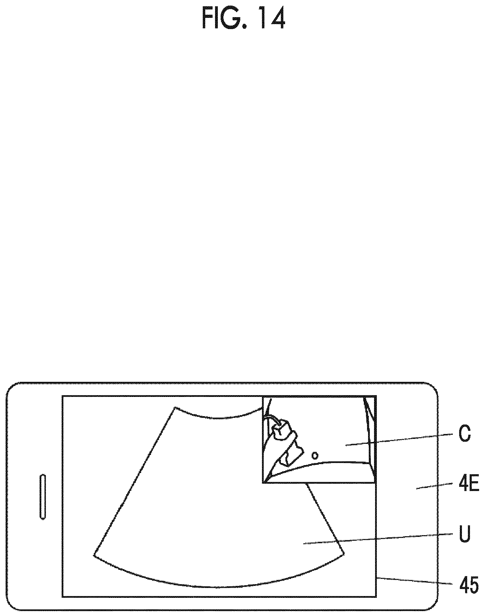

Here, the disposition and the size of the ultrasound image U and the view image C displayed on the external monitor 45 can be adjusted by an input operation of the observer through the input device 47. For example, in a case where the observer inputs instruction information for the guidance on adjusting the disposition and the size of the ultrasound image U and the view image C on the external monitor 45 through the input device 47, the input instruction information is input to the display controller 44 by way of the external controller 46E. The display controller 44 displays the ultrasound image U and the view image C synchronized with each other, for example, with the disposition and the size as shown in Fig. 14 based on the input instruction information. In an example shown in Fig. 14, the external apparatus 4E, the ultrasound image U, and the view image C are rotated at 90 degrees, and the ultrasound image U and the view image C are displayed on the external monitor 45 such that the view image C is superimposed on a part of the ultrasound image U, compared to the example shown in Fig. 5.

From the above description, with the ultrasound system 1E according to Embodiment 5 of the present invention, the disposition and the size of the ultrasound image U and the view image C displayed on the external monitor 45 of the external apparatus 4E can be adjusted. Thus, the observer who observes the ultrasound image U and the view image C displayed on the external monitor 45 can more clearly confirm the ultrasound image U and the view image C conforming to the observer's preference.

### Embodiment 6

In Embodiment 5, although the reception data before imaging subjected to the envelope detection processing by the signal processing unit 23 is wirelessly transmitted to the HMD 3E by the probe-side wireless communication unit 24, the ultrasound image U may be generated in the ultrasound probe 2.

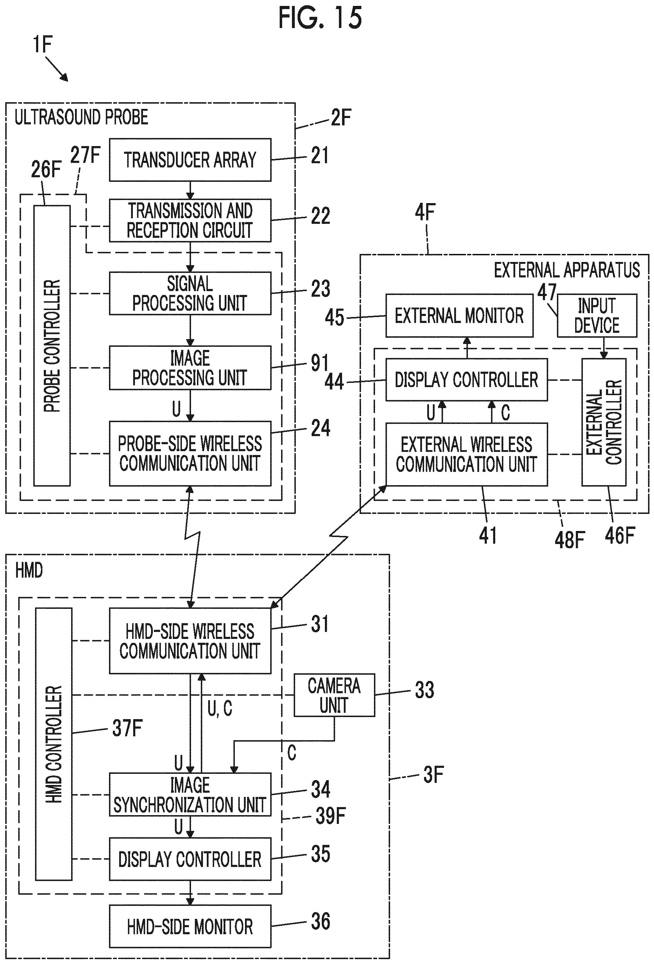

Fig. 15 shows the configuration of an ultrasound system 1F according to Embodiment 6 of the present invention. The ultrasound system 1F comprises an ultrasound probe 2F instead of the ultrasound probe 2E, comprises an HMD 3F instead of the HMD 3E, and comprises an external apparatus 4F instead of the external apparatus 4E, compared to the ultrasound system 1E according to Embodiment 5 shown in Fig. 13. The ultrasound probe 2F is connected only to the HMD 3F by wireless communication, and the external apparatus 4F is connected only to the HMD 3F by wireless communication.

The ultrasound probe 2F is further provided with an image processing unit 91, comprises a probe controller 26F instead of the probe controller 26, and comprises a probe-side processor 27F instead of the probe-side processor 27, compared to the ultrasound probe 2E in Embodiment 5. In the ultrasound probe 2F, the image processing unit 91 is connected to the signal processing unit 23, and the probe-side wireless communication unit 24 and the probe controller 26F are connected to the image processing unit 91. Though not shown, the signal processing unit 23 and the image processing unit 91 configure an ultrasound image generation unit.

The HMD 3F is not provided with the image processing unit 32, comprises an HMD controller 37F instead of the HMD controller 37E, and comprises an HMD-side processor 39F instead of the HMD-side processor 39E, compared to the HMD 3E in Embodiment 5. In the HMD 3F, the image synchronization unit 34 is connected to the HMD-side wireless communication unit 31. The camera unit 33 is connected to the image synchronization unit 34.

The external apparatus 4F comprises an external controller 46F instead of the external controller 46E and comprises an external apparatus-side processor 48F instead of the external apparatus-side processor 48E, compared to the external apparatus 4E in Embodiment 5.

The image processing unit 91 of the ultrasound probe 2F raster-converts the signal subjected to the envelope detection processing by the signal processing unit 23 into an image signal conforming to a normal television signal scanning system and executes various kinds of necessary image processing, such as brightness correction, gradation correction, sharpness correction, an image size correction, refresh rate correction, scanning frequency correction, and color correction, on the converted image signal, thereby generating an ultrasound image U conforming to the display format for the HMD-side monitor 36 of the HMD 3F and an ultrasound image U conforming to a display format for the external monitor 45 of the external apparatus 4F. The image processing unit 91 sends the generated ultrasound images U to the probe-side wireless communication unit 24.

The HMD-side wireless communication unit 31 receives the ultrasound image U wirelessly transmitted from the ultrasound probe 2F. The HMD-side wireless communication unit 31 sends the ultrasound image U conforming to the display format for the HMD-side monitor 36 to the display controller 35 by way of the image synchronization unit 34 and sends the ultrasound image U conforming to the display format for the external monitor 45 to the image synchronization unit 34.

The display controller 35 executes predetermined processing on the ultrasound image U sent from the HMD-side wireless communication unit 31 by way of the image synchronization unit 34 and displays the ultrasound image U on the HMD-side monitor 36 as shown in Fig. 4.

The camera unit 33 acquires a view image C obtained by imaging a scanning point of the ultrasound probe 2F in the subject P and sends the acquired view image C to the image synchronization unit 34.

The image synchronization unit 34 synchronizes the ultrasound image U sent from the HMD-side wireless communication unit 31 and the view image C sent from the camera unit 33 with each other.

The image synchronization unit 34 sends each of the ultrasound image U and the view image C to the HMD-side wireless communication unit 31, instead of generating one composite image M based on the ultrasound image U conforming to the display format for the external monitor 45 and the view image C synchronized with each other.

The HMD-side wireless communication unit 31 wirelessly transmits the ultrasound image U and the view image C sent from the image synchronization unit 34 to the external apparatus 4F.

The external wireless communication unit 41 of the external apparatus 4F receives the ultrasound image U and the view image C wirelessly transmitted from the HMD 3F and sends each of the received ultrasound image U and view image C to the display controller 44.

The display controller 44 executes predetermined processing on the ultrasound image U and the view image C sent from the external wireless communication unit 41 and displays the ultrasound image U and the view image C synchronized with each other together on the external monitor 45.

In this case, the display controller 44 can adjust the disposition and the size of the ultrasound image U and the view image C displayed on the external monitor 45 in response to an input operation of the observer through the input device 47. With this, for example, as shown in Fig. 14, the ultrasound image U and the view image C synchronized with each other are displayed together on the external monitor 45.

From the above description, with the ultrasound system 1F according to Embodiment 6 of the present invention, even in a case where the ultrasound probe 2F comprises the image processing unit 91, the disposition and the size of the ultrasound image U and the view image C displayed on the external monitor 45 of the external apparatus 4F can be adjusted. Thus, the observer who observes the ultrasound image U and the view image C displayed on the external monitor 45 can more clearly confirm the ultrasound image U and the view image C conforming to the observer's preference.

### Explanation of References

1, 1B, 1C, 1D, 1E, 1F: ultrasound system
2, 2B, 2C, 2D, 2E, 2F: ultrasound probe
3, 3A, 3B, 3C, 3D, 3E, 3F: HMD
4, 4A, 4B, 4C, 4D, 4E, 4F: external apparatus
21: transducer array
22: transmission and reception circuit
23: signal processing unit
24: probe-side wireless communication unit
26, 26B, 26D, 26F: probe controller
27, 27B, 27D, 27F: probe-side processor
31: HMD-side wireless communication unit
32, 42, 71, 81, 91: image processing unit
33: camera unit
34, 43: image synchronization unit
35, 44: display controller
36: HMD-side monitor
36A, 36B: monitor
37, 37A, 37B, 37C, 37D, 37E, 37F: HMD controller
39, 39A, 39B, 39C, 39D, 39E, 39F: HMD-side processor
41: external wireless communication unit
45: external monitor
46, 46A, 46B, 46C, 46D, 46E, 46F: external controller
47: input device
48, 48A, 48B, 48C, 48D, 48E, 48F: external apparatus-side processor
51: pulser
52: amplification unit
53: AD conversion unit
54: beamformer
61, 63: microphone
62, 64: speaker
A: cursor
B: bridge portion
C: view image
D: accommodation portion
F: imaging lens
P: subject
T: temple portion
U: ultrasound image

## Claims

1. An ultrasound system (1B) comprising:
an ultrasound probe (2B);
a head-mounted display (3B); and
an external apparatus (4B),
wherein the ultrasound probe (2B) includes
a transducer array (21),
a transmission and reception circuit (22) that transmits an ultrasonic wave from the transducer array (21) and generates a sound ray signal based on a reception signal acquired by the transducer array (21),
an ultrasound image generation unit that generates an ultrasound image based on the sound ray signal generated by the transmission and reception circuit (22), and
a probe-side wireless communication unit (24) that wirelessly transmits the ultrasound image,
the head-mounted display (3B) includes
a camera unit (33) that acquires a view image obtained by imaging a scanning point of the ultrasound probe (2B) in a subject, and
a head-mounted display-side wireless communication unit (31) that wirelessly transmits the view image acquired by the camera unit (33), and
the external apparatus (4B) includes
an external wireless communication unit (41) that is wirelessly connected to at least the head-mounted display-side wireless communication unit (31),
a first image synchronization unit (43) that synchronizes the ultrasound image wirelessly transmitted from the ultrasound probe (2B) and the view image wirelessly transmitted from the head-mounted display (3B) captured at the same timing with each other by referring to a time stamp of the ultrasound image representing a time at which the ultrasound image is captured and a time stamp of the view image representing a time at which the view image is captured,
an external monitor (45), and
a display controller (44) that displays the ultrasound image and the view image synchronized with each other by the first image synchronization unit (43) on the external monitor (45).

2. The ultrasound system (1B) according to claim 1,
wherein the external wireless communication unit (41) is wirelessly connected to both the probe-side wireless communication unit (24) and the head-mounted display-side wireless communication unit (31), and
the probe-side wireless communication unit (24) wirelessly transmits the ultrasound image to both the head-mounted display (3B) and the external apparatus (4B).

3. The ultrasound system (1B) according to claim 1,
wherein the probe-side wireless communication unit (24) wirelessly transmits the ultrasound image to the head-mounted display (3B), and
the head-mounted display-side wireless communication unit (31) wirelessly transmits the ultrasound image wirelessly transmitted from the probe-side wireless communication unit (24) and the view image acquired by the camera unit (33) to the external apparatus (4B).

4. The ultrasound system (1B) according to claim 2 or 3,
wherein the head-mounted display (3B) includes a head-mounted display-side monitor (36), and
the ultrasound image is displayed on the head-mounted display-side monitor (36).

5. The ultrasound system (1B) according to claim 4,
wherein the head-mounted display (3B) includes a second image synchronization unit that synchronizes the ultrasound image and the view image with each other.

6. The ultrasound system (1B) according to claim 4 or 5,
wherein the external apparatus (4B) includes an input device (47),
the external wireless communication unit (41) wirelessly transmits external input information input through the input device (47), to the head-mounted display-side wireless communication unit (31), and
the external input information is displayed on the head-mounted display-side monitor (36).

7. The ultrasound system (1B) according to any one of claims 1 to 6,
wherein wireless communication of voice data is performed between the head-mounted display-side wireless communication unit (31) and the external wireless communication unit (41) in two directions.

8. A method of controlling an ultrasound system (1B) including an ultrasound probe (2B), a head-mounted display (3B), and an external apparatus (4B), the method comprising:
at the ultrasound probe (2B),
transmitting an ultrasonic wave from the transducer array (21) of the ultrasound probe (2B) and generating a sound ray signal based on a reception signal acquired by the transducer array (21),
generating an ultrasound image based on the generated sound ray signal, and
wirelessly transmitting the ultrasound image;
at the head-mounted display (3B),
acquiring a view image obtained by imaging a scanning point of the ultrasound probe (2B) in a subject, and
wirelessly transmitting the acquired view image; and
at the external apparatus (4B),
synchronizing the ultrasound image wirelessly transmitted from the ultrasound probe (2B) and the view image wirelessly transmitted from the head-mounted display (3B) captured at the same timing with each other by referring to a time stamp of the ultrasound image representing a time at which the ultrasound image is captured and a time stamp of the view image representing a time at which the view image is captured,
displaying the ultrasound image and view image synchronized with each other on the external monitor (45).

9. An ultrasound system (1) comprising:
an ultrasound probe (2);
a head-mounted display (3); and
an external apparatus (4),
wherein the ultrasound probe (2) includes
a transducer array (21),
a transmission and reception circuit (22) that transmits an ultrasonic wave from the transducer array (21) and generates a sound ray signal based on a reception signal acquired by the transducer array (21),
a reception data generation unit that generates reception data before imaging by executing signal processing on the sound ray signal generated by the transmission and reception circuit (22), and
a probe-side wireless communication unit (24) that wirelessly transmits the reception data,
the head-mounted display (3) includes
a camera unit (33) that acquires a view image obtained by imaging a scanning point of the ultrasound probe (2) in a subject, and
a head-mounted display-side wireless communication unit (31) that wirelessly transmits the view image acquired by the camera unit (33), and
the external apparatus (4) includes
an external wireless communication unit (41) that is wirelessly connected to at least the head-mounted display-side wireless communication unit (31),
a first image synchronization unit (43) that synchronizes an ultrasound image which is generated based on the reception data wirelessly transmitted from the ultrasound probe (2) and the view image wirelessly transmitted from the head-mounted display (3) captured at the same timing with each other by referring to a time stamp of the ultrasound image representing a time at which the ultrasound image is captured and a time stamp of the view image representing a time at which the view image is captured,
an external monitor (45), and
a display controller (44) that displays the ultrasound image and the view image synchronized with each other by the first image synchronization unit (43) on the external monitor (45).

10. The ultrasound system (1) according to claim 9,
wherein the external wireless communication unit (41) is wirelessly connected to both the probe-side wireless communication unit (24) and the head-mounted display-side wireless communication unit (31), and
the probe-side wireless communication unit (24) wirelessly transmits the reception data to both the head-mounted display (3) and the external apparatus (4).

11. The ultrasound system (1) according to claim 9,
wherein the probe-side wireless communication unit (24) wirelessly transmits the reception data to the head-mounted display (3), and
the head-mounted display-side wireless communication unit (31) wirelessly transmits the reception data wirelessly transmitted from the probe-side wireless communication unit (24) and the view image acquired by the camera unit (33) to the external apparatus (4).

12. The ultrasound system (1) according to claim 10 or 11,
wherein the external apparatus (4) includes an image processing unit (42) that generates the ultrasound image based on the reception data wirelessly transmitted from the probe-side wireless communication unit (24).

13. The ultrasound system (1) according to claim 9,
wherein the probe-side wireless communication unit (24) wirelessly transmits the reception data to the head-mounted display (3),
the head-mounted display (3) includes an image processing unit that generates the ultrasound image based on the reception data wirelessly transmitted from the probe-side wireless communication unit (24), and
the head-mounted display-side wireless communication unit (31) wirelessly transmits the ultrasound image generated by the image processing unit and the view image acquired by the camera unit (33) to the external apparatus (4).

14. The ultrasound system (1) according to claim 13,
wherein the head-mounted display (3) includes a head-mounted display-side monitor (36), and
the ultrasound image is displayed on the head-mounted display-side monitor (36).

15. The ultrasound system (1) according to claim 14,
wherein the head-mounted display (3) includes a second image synchronization unit that synchronizes the ultrasound image and the view image with each other.

16. The ultrasound system (1) according to claim 14 or 15,
wherein the external apparatus (4) includes an input device (47),
the external wireless communication unit (41) wirelessly transmits external input information input through the input device (47), to the head-mounted display-side wireless communication unit (31), and
the external input information is displayed on the head-mounted display-side monitor (36).

17. The ultrasound system (1) according to any one of claims 9 to 16,
wherein wireless communication of voice data is performed between the head-mounted display-side wireless communication unit (31) and the external wireless communication unit (41) in two directions.

18. A method of controlling an ultrasound system (1) including an ultrasound probe (2), a head-mounted display (3), and an external apparatus (4), the method comprising:
at the ultrasound probe (2),
transmitting an ultrasonic wave from the transducer array (21) of the ultrasound probe (2) and generating a sound ray signal based on a reception signal acquired by the transducer array (21),
generating reception data before imaging by executing signal processing on the generated sound ray signal, and
wirelessly transmitting the reception data;
at the head-mounted display (3),
acquiring a view image obtained by imaging a scanning point of the ultrasound probe (2) in a subject, and
wirelessly transmitting the acquired view image; and
at the external apparatus (4),
synchronizing an ultrasound image which is generated based on the reception data wirelessly transmitted from the ultrasound probe (2) and the view image wirelessly transmitted from the head-mounted display (3) captured at the same timing with each other by referring to a time stamp of the ultrasound image representing a time at which the ultrasound image is captured and a time stamp of the view image representing a time at which the view image is captured,
displaying the ultrasound image and the view image synchronized with each other on the external monitor (45).

## Patentansprüche

1. Ein Ultraschallsystem (1B), umfassend:
eine Ultraschallsonde (2B);
eine am Kopf anzubringende bzw. Kopf-montierte Anzeige (3B); und
eine externe Vorrichtung (4B),
wobei die Ultraschallsonde (2B) es aufweist
eine Wandleranordnung (21),
eine Sende- und Empfangsschaltung (22), die eine Ultraschallwelle von der Wandleranordnung (21) sendet und ein Schallstrahlsignal auf der Grundlage eines von der Wandleranordnung (21) erfassten Empfangssignals erzeugt,
eine Ultraschallbilderzeugungseinheit, die ein Ultraschallbild auf der Grundlage des von der Sende- und Empfangsschaltung (22) erzeugten Schallstrahlsignals erzeugt, und
eine sondenseitige drahtlose Kommunikationseinheit (24), die das Ultraschallbild drahtlos überträgt,
die Kopf-montierte Anzeige (3B) umfasst
eine Kameraeinheit (33), die ein Ansichtsbild erfasst, das durch Abbilden eines Abtastpunkts der Ultraschallsonde (2B) in einem Subjekt erhalten wird, und
eine drahtlose Kommunikationseinheit (31) auf der Seite der Kopf-montierten Anzeige, die das von der Kameraeinheit (33) erfasste Ansichtsbild drahtlos überträgt, und
die externe Vorrichtung (4B) umfasst
eine externe drahtlose Kommunikationseinheit (41), die drahtlos mit mindestens der drahtlosen Kommunikationseinheit (31) auf der Seite der Kopf-montierten Anzeige (31) verbunden ist,
eine erste Bildsynchronisationseinheit (43), die das von der Ultraschallsonde (2B) drahtlos übertragene Ultraschallbild und das von der am Kopf montierten Anzeige (3B) drahtlos übertragene Ansichtsbild, die zum gleichen Zeitpunkt aufgenommen wurden, miteinander synchronisiert, indem sie sich auf einen Zeitstempel des Ultraschallbildes, der einen Zeitpunkt darstellt, zu dem das Ultraschallbild aufgenommen wurde, und einen Zeitstempel des Ansichtsbildes, der einen Zeitpunkt darstellt, zu dem das Ansichtsbild aufgenommen wurde, bezieht,
einen externen Monitor (45), und
eine Anzeigesteuerung (44), die das Ultraschallbild und das Ansichtsbild, die durch die erste Bildsynchronisationseinheit (43) miteinander synchronisiert sind, auf dem externen Monitor (45) anzeigt.

2. Das Ultraschallsystem (1B) nach Anspruch 1,
wobei die externe drahtlose Kommunikationseinheit (41) drahtlos sowohl mit der sondenseitigen drahtlosen Kommunikationseinheit (24) als auch mit der drahtlosen Kommunikationseinheit (31) auf der Seite der Kopf-montierten Anzeige verbunden ist, und
die sondenseitige drahtlose Kommunikationseinheit (24) das Ultraschallbild drahtlos sowohl an die am Kopf montierte Anzeige (3B) als auch an die externe Vorrichtung (4B) überträgt.

3. Das Ultraschallsystem (1B) nach Anspruch 1,
wobei die sondenseitige drahtlose Kommunikationseinheit (24) das Ultraschallbild drahtlos an die am Kopf montierte Anzeige (3B) überträgt, und
die drahtlose Kommunikationseinheit (31) auf der Seite der Kopf-montierten Anzeige das von der sondenseitigen drahtlosen Kommunikationseinheit (24) drahtlos übertragene Ultraschallbild und das von der Kameraeinheit (33) erfasste Ansichtsbild drahtlos an die externe Vorrichtung (4B) überträgt.

4. Das Ultraschallsystem (1B) nach Anspruch 2 oder 3,
wobei die Kopf-montierte Anzeige (3B) einen Kopf-montierten anzeigenseitigen Monitor (36) umfasst, und
das Ultraschallbild auf dem kopfgetragenen Monitor (36) angezeigt wird.

5. Das Ultraschallsystem (1B) nach Anspruch 4,
wobei die Kopf-montierte Anzeige (3B) eine zweite Bildsynchronisationseinheit enthält, die das Ultraschallbild und das Ansichtsbild miteinander synchronisiert.

6. Das Ultraschallsystem (1B) nach Anspruch 4 oder 5,
wobei die externe Vorrichtung (4B) eine Eingabevorrichtung (47) enthält,
die externe drahtlose Kommunikationseinheit (41) externe Eingabeinformationen, die über die Eingabevorrichtung (47) eingegeben werden, drahtlos an die kopfseitige drahtlose Kommunikationseinheit (31) überträgt, und
die externen Eingangsinformationen auf dem kopfgetragenen Monitor (36) angezeigt werden.

7. Das Ultraschallsystem (1B) nach einem der Ansprüche 1 bis 6,
wobei die drahtlose Kommunikation von Sprachdaten zwischen der kopfseitigen drahtlosen Kommunikationseinheit (31) und der externen drahtlosen Kommunikationseinheit (41) in zwei Richtungen durchgeführt wird.

8. Verfahren zur Steuerung eines Ultraschallsystems (1B) mit einer Ultraschallsonde (2B), einer am Kopf anzubringenden bzw. Kopf-montierten Anzeige (3B) und einer externen Vorrichtung (4B), wobei das Verfahren Folgendes umfasst:
an der Ultraschallsonde (2B),
Senden einer Ultraschallwelle von der Wandleranordnung (21) der Ultraschallsonde (2B) und Erzeugen eines Schallstrahlsignals auf der Grundlage eines von der Wandleranordnung (21) erfassten Empfangssignals,
Erzeugen eines Ultraschallbildes auf der Grundlage des erzeugten Schallstrahlsignals, und
drahtlose Übertragung des Ultraschallbildes;
auf der Kopf-montierten Anzeige (3B),
Erfassen eines Ansichtsbildes, das durch Abbilden eines Abtastpunktes der Ultraschallsonde (2B) in einem Subjekt erhalten wird, und
drahtlose Übertragung des erfassten Ansichtsbildes, und
am externen Gerät (4B),
Synchronisieren des von der Ultraschallsonde (2B) drahtlos übertragenen Ultraschallbildes und des von der Kopf-montierten Anzeige (3B) drahtlos übertragenen Ansichtsbildes, die zur gleichen Zeit aufgenommen wurden, miteinander durch Bezugnahme auf einen Zeitstempel des Ultraschallbildes, der eine Zeit darstellt, zu der das Ultraschallbild aufgenommen wurde, und einen Zeitstempel des Ansichtsbildes, der eine Zeit darstellt, zu der das Ansichtsbild aufgenommen wurde,
Anzeige des Ultraschallbildes und des Ansichtsbildes synchron zueinander auf dem externen Monitor (45).

9. Ein Ultraschallsystem (1), das Folgendes umfasst:
eine Ultraschallsonde (2);
eine Kopf-montierte Anzeige (3) und
eine externe Vorrichtung (4),
wobei die Ultraschallsonde (2) Folgendes umfasst
eine Wandleranordnung (21),
eine Sende- und Empfangsschaltung (22), die eine Ultraschallwelle von der Wandleranordnung (21) sendet und ein Schallstrahlsignal auf der Grundlage eines von der Wandleranordnung (21) erfassten Empfangssignals erzeugt,
eine Empfangsdaten-Erzeugungseinheit, die vor der Bildgebung Empfangsdaten erzeugt, indem sie eine Signalverarbeitung an dem von der Sende- und Empfangsschaltung (22) erzeugten Schallstrahlsignal durchführt, und
eine sondenseitige drahtlose Kommunikationseinheit (24), die die Empfangsdaten drahtlos überträgt,
die Kopf-montierte Anzeige (3) umfasst
eine Kameraeinheit (33), die ein Ansichtsbild erfasst, das durch Abbilden eines Abtastpunkts der Ultraschallsonde (2) in einem Subjekt erhalten wird, und
eine kopfseitige drahtlose Kommunikationseinheit (31), die das von der Kameraeinheit (33) erfasste Ansichtsbild drahtlos überträgt, und
die externe Vorrichtung (4) umfasst
eine externe drahtlose Kommunikationseinheit (41), die drahtlos mit mindestens der drahtlosen Kommunikationseinheit (31) auf der Seite der Kopf-montierten Anzeige verbunden ist,
eine erste Bildsynchronisationseinheit (43), die ein Ultraschallbild, das auf der Grundlage der von der Ultraschallsonde (2) drahtlos übertragenen Empfangsdaten erzeugt wird, und das von der Kopf-montierten Anzeige (3) drahtlos übertragene Ansichtsbild, das zum gleichen Zeitpunkt aufgenommen wird, miteinander synchronisiert, indem sie sich auf einen Zeitstempel des Ultraschallbildes, der einen Zeitpunkt darstellt, zu dem das Ultraschallbild aufgenommen wird, und einen Zeitstempel des Ansichtsbildes, der einen Zeitpunkt darstellt, zu dem das Ansichtsbild aufgenommen wird, bezieht,
einen externen Monitor (45), und
eine Anzeigesteuerung (44), die das Ultraschallbild und das Ansichtsbild, die durch die erste Bildsynchronisationseinheit (43) miteinander synchronisiert wurden, auf dem externen Monitor (45) anzeigt.

10. Das Ultraschallsystem (1) nach Anspruch 9,
wobei die externe drahtlose Kommunikationseinheit (41) drahtlos sowohl mit der sondenseitigen drahtlosen Kommunikationseinheit (24) als auch der drahtlosen Kommunikationseinheit (31) auf der Seite der Kopf-montierten Anzeige verbunden ist, und
die sondenseitige drahtlose Kommunikationseinheit (24) die Empfangsdaten drahtlos sowohl an die am Kopf getragene Anzeige (3) als auch an die externe Vorrichtung (4) überträgt.

11. Das Ultraschallsystem (1) nach Anspruch 9,
wobei die sondenseitige drahtlose Kommunikationseinheit (24) die Empfangsdaten drahtlos an die Kopf-montierte Anzeige (3) überträgt, und
die drahtlose Kommunikationseinheit (31) auf der Seite der Kopf-montierten Anzeige die von der sondenseitigen drahtlosen Kommunikationseinheit (24) drahtlos übertragenen Empfangsdaten und das von der Kameraeinheit (33) erfasste Ansichtsbild drahtlos an die externe Vorrichtung (4) überträgt.

12. Das Ultraschallsystem (1) nach Anspruch 10 oder 11,
wobei die externe Vorrichtung (4) eine Bildverarbeitungseinheit (42) enthält, die das Ultraschallbild auf der Grundlage der von der sondenseitigen drahtlosen Kommunikationseinheit (24) drahtlos übertragenen Empfangsdaten erzeugt.

13. Das Ultraschallsystem (1) nach Anspruch 9,
wobei die sondenseitige drahtlose Kommunikationseinheit (24) die Empfangsdaten drahtlos an die Kopf-montierte Anzeige (3) überträgt,
die Kopf-montierte Anzeige (3) eine Bildverarbeitungseinheit enthält, die das Ultraschallbild auf der Grundlage der von der sondenseitigen drahtlosen Kommunikationseinheit (24) drahtlos übertragenen Empfangsdaten erzeugt, und
die kopfseitige drahtlose Kommunikationseinheit (31) das von der Bildverarbeitungseinheit erzeugte Ultraschallbild und das von der Kameraeinheit (33) aufgenommene Ansichtsbild drahtlos an die externe Vorrichtung (4) überträgt.

14. Das Ultraschallsystem (1) nach Anspruch 13,
wobei die Kopf-montierte Anzeige (3) einen Kopf-montierten bzw. zu tragenden anzeigenseitigen Monitor (36) umfasst, und
das Ultraschallbild auf dem Kopf-montierten Monitor (36) angezeigt wird.

15. Das Ultraschallsystem (1) nach Anspruch 14,
wobei die Kopf-montierte Anzeige (3) eine zweite Bildsynchronisationseinheit aufweist, die das Ultraschallbild und das Ansichtsbild miteinander synchronisiert.

16. Das Ultraschallsystem (1) nach Anspruch 14 oder 15,
wobei die externe Vorrichtung (4) eine Eingabevorrichtung (47) enthält,
die externe drahtlose Kommunikationseinheit (41) externe Eingabeinformationen, die über die Eingabevorrichtung (47) eingegeben werden, drahtlos an die drahtlose Kommunikationseinheit (31) auf der Seite der Kopf-montierten Anzeige überträgt, und
die externen Eingangsinformationen auf dem Kopf-montierten Monitor (36) angezeigt werden.

17. Das Ultraschallsystem (1) nach einem der Ansprüche 9 bis 16,
wobei die drahtlose Kommunikation von Sprachdaten zwischen der drahtlosen Kommunikationseinheit (31) auf der Seite der Kopf-montierten Anzeige und der externen drahtlosen Kommunikationseinheit (41) in zwei Richtungen durchgeführt wird.

18. Verfahren zur Steuerung eines Ultraschallsystems (1) mit einer Ultraschallsonde (2), einer am Kopf montierten Anzeige (3) und einem externen Gerät (4), wobei das Verfahren Folgendes umfasst:
an der Ultraschallsonde (2),
Senden einer Ultraschallwelle von der Wandleranordnung (21) der Ultraschallsonde (2) und Erzeugen eines Schallstrahlsignals auf der Grundlage eines von der Wandleranordnung (21) erfassten Empfangssignals,
Erzeugen von Empfangsdaten vor der Bildgebung durch Ausführen von Signalverarbeitung an dem erzeugten Schallstrahlsignal, und
drahtlose Übermittlung der Empfangsdaten;
auf der Kopf-montierten Anzeige (3),
Erfassen eines Ansichtsbildes, das durch Abbilden eines Abtastpunktes der Ultraschallsonde (2) in einem Subjekt erhalten wird, und
drahtlose Übertragung des erfassten Ansichtsbildes, und
am externen Gerät (4),
Synchronisieren eines Ultraschallbildes, das auf der Grundlage der von der Ultraschallsonde (2) drahtlos übertragenen Empfangsdaten erzeugt wird, und des von der Kopf-montierten Anzeige (3) drahtlos übertragenen Ansichtsbildes, das zum gleichen Zeitpunkt aufgenommen wird, miteinander durch Bezugnahme auf einen Zeitstempel des Ultraschallbildes, der einen Zeitpunkt darstellt, zu dem das Ultraschallbild aufgenommen wird, und einen Zeitstempel des Ansichtsbildes, der einen Zeitpunkt darstellt, zu dem das Ansichtsbild aufgenommen wird,
Anzeige des Ultraschallbildes und des Ansichtsbildes synchron zueinander auf dem externen Monitor (45).

## Revendications

1. Système à ultrasons (1B) comprenant :
une sonde à ultrasons (2B) ;
un affichage monté sur la tête (3B) ; et
un appareil externe (4B),
dans laquelle la sonde à ultrasons (2B) comprend es
un réseau de transducteurs (21),
un circuit de transmission et de réception (22) qui transmet une onde ultrasonique à partir du réseau de transducteurs (21) et génère un signal de rayon sonore sur la base d'un signal de réception acquis par le réseau de transducteurs (21),
une unité de génération d'images ultrasonores qui génère une image ultrasonore sur la base du signal de rayon sonore généré par le circuit de transmission et de réception (22), et
une unité de communication sans fil côté sonde (24) qui transmet sans fil l'image ultrasonore,
l'affichage monté sur la tête (3B) comprend
une unité de caméra (33) qui acquiert une image de vue obtenue par imagerie d'un point de balayage de la sonde à ultrasons (2B) dans un sujet, et
une unité de communication sans fil du côté de l'affichage monté sur la tête (31) qui transmet sans fil l'image acquise par l'unité de caméra (33), et
l'appareil externe (4B) comprend
une unité de communication sans fil externe (41) qui est connectée sans fil à au moins l'unité de communication sans fil du côté de l'affichage monté sur la tête (31),
une première unité de synchronisation d'image (43) qui synchronise l'image ultrasonore transmise sans fil par la sonde ultrasonore (2B) et l'image visuelle transmise sans fil par l'affichage monté sur la tête (3B) capturées au même moment en se référant à un horodatage de l'image ultrasonore représentant le moment où l'image ultrasonore est capturée et à un horodatage de l'image visuelle représentant le moment où l'image visuelle est capturée,
un moniteur externe (45), et
un contrôleur d'affichage (44) qui affiche sur le moniteur externe (45) l'image ultrasonore et l'image de la vue synchronisées l'une avec l'autre par la première unité de synchronisation d'image (43).

2. Système à ultrasons (1B) selon la revendication 1,
dans lequel l'unité de communication sans fil externe (41) est connectée sans fil à l'unité de communication sans fil côté sonde (24) et à l'unité de communication sans fil côté affichage monté sur la tête (31), et
l'unité de communication sans fil côté sonde (24) transmet sans fil l'image ultrasonore à l'affichage monté sur la tête (3B) et à l'appareil externe (4B).

3. Système à ultrasons (1B) selon la revendication 1,
l'unité de communication sans fil côté sonde (24) transmet sans fil l'image ultrasonore à l'écran monté sur la tête (3B), et
l'unité de communication sans fil du côté de l'affichage monté sur la tête (31) transmet sans fil l'image ultrasonore transmise sans fil par l'unité de communication sans fil du côté de la sonde (24) et l'image acquise par l'unité de caméra (33) à l'appareil externe (4B).

4. Système à ultrasons (1B) selon la revendication 2 ou 3,
dans lequel l'affichage monté sur la tête (3B) comprend un moniteur latéral d'affichage monté sur la tête (36), et
l'image échographique est affichée sur l'écran latéral monté sur la tête (36).

5. Système à ultrasons (1B) selon la revendication 4,
l'affichage monté sur la tête (3B) comprend une deuxième unité de synchronisation d'image qui synchronise l'image ultrasonore et l'image de la vue l'une avec l'autre.

6. Système à ultrasons (1B) selon la revendication 4 ou 5,
dans lequel l'appareil externe (4B) comprend un dispositif d'entrée (47),
l'unité de communication externe sans fil (41) transmet sans fil à l'unité de communication sans fil du côté de l'affichage monté sur la tête (31) les informations d'entrée externe saisies par l'intermédiaire du dispositif d'entrée (47), et
les informations de l'entrée externe sont affichées sur l'écran latéral de la tête de lecture (36).

7. Système à ultrasons (1B) selon l'une des revendications 1 à 6,
dans lequel la communication sans fil de données vocales est effectuée entre l'unité de communication sans fil (31) du côté de l'affichage monté sur la tête et l'unité de communication sans fil externe (41) dans deux directions.

8. Méthode de commande d'un système à ultrasons (1B) comprenant une sonde à ultrasons (2B), un affichage monté sur la tête (3B) et un appareil externe (4B), la méthode comprenant :
au niveau de la sonde à ultrasons (2B),
transmettre une onde ultrasonique à partir du réseau de transducteurs (21) de la sonde à ultrasons (2B) et générer un signal de rayon sonore sur la base d'un signal de réception acquis par le réseau de transducteurs (21),
générer une image ultrasonore sur la base du signal de rayon sonore généré, et
transmission sans fil de l'image ultrasonore ;
sur l'affichage monté sur la tête (3B),
acquisition d'une image de vue obtenue par imagerie d'un point de balayage de la sonde à ultrasons (2B) dans un sujet, et
transmission sans fil de l'image acquise ; et
au niveau de l'appareil externe (4B),
synchroniser l'image ultrasonore transmise sans fil par la sonde ultrasonore (2B) et l'image visuelle transmise sans fil par l'affichage monté sur la tête (3B) capturées au même moment en se référant à un horodatage de l'image ultrasonore représentant un moment où l'image ultrasonore est capturée et un horodatage de l'image visuelle représentant un moment où l'image visuelle est capturée,
afficher l'image échographique et l'image visuelle synchronisées l'une avec l'autre sur le moniteur externe (45).

9. Système à ultrasons (1) comprenant :
une sonde à ultrasons (2) ;
un affichage monté sur la tête (3) ; et
un appareil externe (4),
dans laquelle la sonde à ultrasons (2) comprend
un réseau de transducteurs (21),
un circuit de transmission et de réception (22) qui transmet une onde ultrasonique à partir du réseau de transducteurs (21) et génère un signal de rayon sonore sur la base d'un signal de réception acquis par le réseau de transducteurs (21),
une unité de génération de données de réception qui génère des données de réception avant l'imagerie en exécutant un traitement de signal sur le signal de rayon sonore généré par le circuit de transmission et de réception (22), et
une unité de communication sans fil côté sonde (24) qui transmet sans fil les données de réception,
l'affichage monté sur la tête (3) comprend
une unité de caméra (33) qui acquiert une image de vue obtenue par imagerie d'un point de balayage de la sonde à ultrasons (2) dans un sujet, et
une unité de communication sans fil du côté de l'affichage monté sur la tête (31) qui transmet sans fil l'image acquise par l'unité de caméra (33), et
l'appareil externe (4) comprend
une unité de communication sans fil externe (41) qui est connectée sans fil à au moins l'unité de communication sans fil du côté de l'affichage monté sur la tête (31),
une première unité de synchronisation d'image (43) qui synchronise une image ultrasonique générée sur la base des données de réception transmises sans fil par la sonde ultrasonique (2) et l'image visuelle transmise sans fil par l'affichage monté sur la tête (3) capturées au même moment l'une avec l'autre en se référant à un horodatage de l'image ultrasonique représentant un moment où l'image ultrasonique est capturée et un horodatage de l'image visuelle représentant un moment où l'image visuelle est capturée,
un moniteur externe (45), et
un contrôleur d'affichage (44) qui affiche sur le moniteur externe (45) l'image ultrasonore et l'image de la vue synchronisées l'une avec l'autre par la première unité de synchronisation d'image (43).

10. Système à ultrasons (1) selon la revendication 9,
dans lequel l'unité de communication sans fil externe (41) est connectée sans fil à l'unité de communication sans fil côté sonde (24) et à l'unité de communication sans fil côté affichage monté sur la tête (31), et
l'unité de communication sans fil côté sonde (24) transmet sans fil les données de réception à la fois à l'affichage monté sur la tête (3) et à l'appareil externe (4).

11. Système à ultrasons (1) selon la revendication 9,
l'unité de communication sans fil côté sonde (24) transmet sans fil les données de réception à l'affichage monté sur la tête (3), et
l'unité de communication sans fil du côté de l'affichage monté sur la tête (31) transmet sans fil les données de réception transmises sans fil par l'unité de communication sans fil du côté de la sonde (24) et l'image acquise par l'unité de caméra (33) à l'appareil externe (4).

12. Système à ultrasons (1) selon la revendication 10 ou 11,
l'appareil externe (4) comprend une unité de traitement d'images (42) qui génère l'image ultrasonore sur la base des données de réception transmises sans fil par l'unité de communication sans fil côté sonde (24).

13. Système à ultrasons (1) selon la revendication 9,
l'unité de communication sans fil côté sonde (24) transmet sans fil les données de réception à l'affichage monté sur la tête (3),
l'affichage monté sur la tête (3) comprend une unité de traitement d'image qui génère l'image ultrasonore sur la base des données de réception transmises sans fil par l'unité de communication sans fil côté sonde (24), et
l'unité de communication sans fil côté écran monté sur la tête (31) transmet sans fil à l'appareil externe (4) l'image ultrasonore générée par l'unité de traitement d'images et l'image de la vue acquise par l'unité de caméra (33).

14. Système à ultrasons (1) selon la revendication 13,
dans lequel l'affichage monté sur la tête (3) comprend un moniteur latéral d'affichage monté sur la tête (36), et
l'image échographique est affichée sur l'écran latéral monté sur la tête (36).

15. Système à ultrasons (1) selon la revendication 14,
l'affichage monté sur la tête (3) comprend une deuxième unité de synchronisation d'image qui synchronise l'image ultrasonore et l'image de la vue l'une avec l'autre.

16. Système à ultrasons (1) selon la revendication 14 ou 15,
dans lequel l'appareil externe (4) comprend un dispositif d'entrée (47),
l'unité de communication externe sans fil (41) transmet sans fil à l'unité de communication sans fil du côté de l'affichage monté sur la tête (31) les informations d'entrée externe saisies par l'intermédiaire du dispositif d'entrée (47), et
les informations de l'entrée externe sont affichées sur l'écran latéral de la tête de lecture (36).

17. Système à ultrasons (1) selon l'une des revendications 9 à 16,
dans lequel la communication sans fil de données vocales est effectuée entre l'unité de communication sans fil (31) du côté de l'affichage monté sur la tête et l'unité de communication sans fil externe (41) dans deux directions.

18. Méthode de contrôle d'un système à ultrasons (1) comprenant une sonde à ultrasons (2), un affichage monté sur la tête (3) et un appareil externe (4), la méthode comprenant :
à la sonde à ultrasons (2),
transmettre une onde ultrasonique à partir du réseau de transducteurs (21) de la sonde à ultrasons (2) et générer un signal de rayon sonore sur la base d'un signal de réception acquis par le réseau de transducteurs (21),
générer des données de réception avant l'imagerie en exécutant un traitement de signal sur le signal de rayon sonore généré, et
transmettre sans fil les données de réception ;
sur l'affichage monté sur la tête (3),
acquisition d'une image de vue obtenue par imagerie d'un point de balayage de la sonde à ultrasons (2) dans un sujet, et
transmission sans fil de l'image acquise ; et
au niveau de l'appareil externe (4),
synchroniser une image ultrasonore générée sur la base des données de réception transmises sans fil par la sonde ultrasonore (2) et l'image visuelle transmise sans fil par l'affichage monté sur la tête (3) capturée au même moment en se référant à un horodatage de l'image ultrasonore représentant le moment où l'image ultrasonore est capturée et à un horodatage de l'image visuelle représentant le moment où l'image visuelle est capturée,
afficher l'image échographique et l'image visuelle synchronisées l'une avec l'autre sur le moniteur externe (45).
